# EUROPÄISCHE PATENTSCHRIFT

(11) **EP 4 326 187 B1**
(45) Veröffentlichungstag und Bekanntmachung des Hinweises auf die Patenterteilung: **08.10.2025**
(21) Anmeldenummer: 22761561.4
(22) Anmeldetag: 10.08.2022
(51) Int. Cl.: A61C 13/00, A61C 13/271

(54) **ROHLING FÜR EINE ZAHNPROTHESE UND VERFAHREN ZU SEINER HERSTELLUNG, VERWENDUNG DES ROHLINGS UND VERFAHREN ZUR HERSTELLUNG EINER ZAHNPROTHESE AUS DEM ROHLING**
BLANK FOR DENTAL PROSTHESIS AND METHOD FOR ITS MANUFACTURE, USE OF THE BLANK, AND METHOD FOR MANUFACTURING A DENTAL RESTORATION FROM THE BLANK
ÉBAUCHE DE PROTHÈSE DENTAIRE ET SON PROCÉDÉ DE FABRICATION, UTILISATION DE L'ÉBAUCHE ET PROCÉDÉ DE FABRICATION D'UNE RESTAURATION DENTAIRE À PARTIR DE L'ÉBAUCHE

(30) Priorität: 10.08.2021 EP 21190668
(43) Veröffentlichungstag der Anmeldung: 28.02.2024
(73) Patentinhaber: Ivoclar Vivadent AG, 9494 Schaan (LI)
(72) Erfinder: PULIDO, Jose, 9443 Widnau (CH); ROTHBRUST, Frank, 6822 Röns (AT); SERBAN, Corina, 88131 Lindau (DE); KROLIKOWSKI, Sebastian, 8853 Lachen (CH); CADUFF, Roman, 7417 Paspels (CH); RITZBERGER, Christian, 9472 Grabs (CH)
(74) Vertreter: Uexküll & Stolberg
(86) Internationale Anmeldenummer: PCT/EP2022/072435
(87) Internationale Veröffentlichungsnummer: WO 2023/017075

(56) Entgegenhaltungen:
- EP-A1- 3 397 193
- EP-B1- 3 397 193
- DE-A1- 102006 010 665
- US-A- 5 939 211
- US-A1- 2014 087 327
- US-A1- 2016 089 221
- US-A1- 2018 071 063
- US-A1- 2020 015 944
- US-A1- 2021 128 283

## Beschreibung

Die Erfindung betrifft einen Rohling für dentale Zwecke, insbesondere zur Herstellung von Dentalrestaurationen wie Dentalprothesen, mit dem die optischen Eigenschaften von natürlichem Zahnmaterial und der natürlichen Mundschleimhaut sehr gut nachgeahmt werden können und der sich aufgrund seiner Eigenschaften insbesondere für die einfache Herstellung von ästhetisch anspruchsvollen Dentalprothesen, wie Oberkiefer-Vollprothesen und/oder Unterkiefer-Vollprothesen, mit sehr guten mechanischen Eigenschaften eignet.

Im Dentalbereich werden heutzutage vermehrt vielgliedrige und teils weitspannige Implantat-gestützte Restaurationen zur Versorgung von Patienten im Unter- und Oberkiefer hergestellt. Neben der Ästhetik der Zähne spielt hierbei auch die farbliche Anpassung des Übergangs zur Mundschleimhaut (Gingiva) und die optimale farbliche Imitierung der eigentlichen Mundschleimhaut eine wesentliche Rolle für das ästhetische Gesamtergebnis. Es ist jedoch ein hoher, manueller Aufwand erforderlich, um den Gingiva-Bereich naturgetreu zu restaurieren.

Vollprothesen wurden in der Vergangenheit üblicherweise hergestellt, indem eine Silikonabformung des unbezahnten Patientenkiefers durchgeführt wird; auf dieser Basis ein Gipsmodell hergestellt wird; unter Zuhilfenahme einer Wachsplatte eine künstliche Zahnreihe auf dem Gipsmodell aufgestellt wird; eine Einprobe beim Patienten und ggf. eine Nachkorrektur durchgeführt werden; die künstlichen Zähne, die Wachsmodellation und ein Teil des Gipsmodells in Gips eingebettet werden; das Wachs ausgeschmolzen wird; der entstandene Hohlraum mit einem PMMAbasierten Prothesenwerkstoff ausgefüllt wird; die Gipseinbettung entfernt; und eine abschließenden Reinigung und Oberflächenpolitur durchgeführt wird. Dieses Verfahren führt in der Regel zu sehr guten Resultaten, ist jedoch sehr zeit- und arbeitsaufwendig.

Mehrfarbige Blöcke, die den Verlauf der Farbe und der Transluzenz vom Dentin zum Zahnschmelz simulieren, und deren Einsatz in der Dentaltechnik sind aus dem Stand der Technik bekannt. Rohlinge auf Basis von Zirkonoxidkeramik und deren Verwendung zur Herstellung von Dentalprothesen in einem CAD/CAM-Verfahren mittels einer computergesteuerten Fräsanlage sind ebenfalls bereits aus dem Stand der Technik bekannt. Allerdings ist auch bei der Verwendung von solchen Rohlingen ein hoher, manueller Aufwand erforderlich, um den Gingiva-Bereich naturgetreu zu restaurieren. Das liegt im Wesentlichen daran, dass gemäß einer ersten Alternative der Gingiva-Bereich nachträglich aufgebracht werden muss, z.B. indem der Anteil der Gingiva durch Aufschichten oder Anpressen mittels Gingiva-farbenen Pressrohlingen an eine ZrO₂-Restauration aufgebrannt oder aufgepresst und mittels Vollkeramik-Techniken naturgetreu nachgestellt wird. Es ist auch möglich, den Gingiva-Bereich nach Fertigstellung der Restauration mittels Kompositmaterial anzuschichten. Hierbei besteht jedoch das Risiko einer Farbinstabilität und Plaque- bzw. Geruchsbildung.

Gemäß einer zweiten Alternative wird ein Zirkonoxidrohling mit vorgeformtem Gingiva-Bereich verwendet, der jedoch noch einer Nachbehandlung bedarf, um eine optimale farbliche Imitierung der Gingiva zu erreichen. Bei Verwendung von zahnfarbenen, voreingefärbten Zirkonoxid-Scheiben mit vorgeformtem Gingiva-Anteil wird der zahnfarben eingefärbte Bereich mit glaskeramischen Schicht-, Verblend-, Mal- und Charakterisierungsmassen in mehreren Schritten überdeckt. Bei Verwendung von nicht eingefärbten oder geringfügig eingefärbten Zirkonoxid-Scheiben mit vorgeformtem Gingiva-Anteil wird das Problem aktuell dadurch gelöst, dass eine hochkonzentrierte Lösung auf Basis von wasserlöslichen Er³⁺-Salzen, wie z.B. ErCl₃, ErCl₃·6H₂O oder Er(NO₃)₃·5H₂O im Gingiva-Bereich infiltriert wird.

Die hochkonzentrierten Infiltrationslösungen auf Er³⁺-Basis weisen einen stark sauren pH-Wert auf, der häufig im Bereich von 1-3 liegt. Aus sicherheitstechnischen Gründen besteht daher bei unsachgemäßem Gebrauch ein sehr großes Risiko, dass sich der Anwender verätzen kann oder schädliche Gase/Dämpfe beim Öffnen der Gebinde ohne Verwendung eines geeigneten Abzugs oder einer adäquaten Raumbelüftung einatmet, wie z.B. HCl- oder HNO₃-Dämpfe. Des Weiteren sind solche Lösungen meist nicht langzeitstabil und verändern daher ihre Eigenschaften im Laufe der Zeit. Auch können sich durch unkontrolliertes Abdampfen des Lösungsmittels über die Zeit Konzentrationsänderungen einstellen. Ebenfalls kann es zu unkontrollierten Komplexbildungen kommen, da diese Lösungen meist auch bestimmte organische Verbindungen enthalten. Dies führt im schlechtesten Falle dazu, dass bestimme Verbindungen ausfallen.

Die Infiltration mit Färbelösung kann ferner dazu führen, dass die Färbelösung nicht homogen im porösen Zirkonoxid verteilt ist. Dies kann sowohl vom Anwender als auch von der Oberflächengüte der Restauration, wie z.B. dem Grad der Staubfreiheit und dem Feuchtigkeitsgehalt, abhängig sein. Inhomogenitäten können einen signifikanten Einfluss auf das Endergebnis haben.

Eine Infiltration im Gingiva-Bereich bewirkt zudem eine Veränderung der Stabilisierungsgrads und der Phasenzusammensetzung des Zirkonoxids nach dem Dichtsintern. Während des Dichtsinterns können zusätzlich zu den bereits im Zirkonoxid eingebauten Y³⁺-Ionen auch Er³⁺-Ionen in das Kristallgerüst eingebaut werden. Dies führt dann zu einer Überstabilisierung und einer damit verbundenen Änderung der Phasenzusammensetzung in den infiltrierten Bereichen. Dies hat eine Verschlechterung von mechanischen Eigenschaften, insbesondere von Bruchzähigkeit und Biaxialfestigkeit, zur Folge. Gerade im Ginigiva-Bereich wird bei Implantat getragenen Restaurationen eine sehr große Kau-Last aufgenommen, so dass eine Schwächung dieses Bereichs zu vermeiden ist.

Darüber hinaus verändert die Dotierung mit Er₂O₃ in höheren Konzentrationen das Sinterverhalten des Zirkonoxids. Er₂O₃ wirkt als Sinter-Aktivator und verändert die theoretische Dichte des stabilisierten Zirkonoxids. Die Gesamtschwindung des infiltrierten Bereichs nimmt zu, so dass der beim Fräsen des Rohlings berücksichtige Vergrößerungsfaktor nicht mehr korrekt ist und die Passgenauigkeit der dichtgesinterten Restauration negativ beeinflusst wird. Wird Er₂O₃ nur lokal zur Einfärbung des Gingiva-Bereichs angewendet, kommt es während der Sinterung lokal zu einem früheren Schrumpfen, d.h. während der Gingiva-Bereich schon schwindet, hängt die Restauration im Zahnbereich der Schwindung hinterher. Dadurch kommt es zu Spannungen während des Sintervorgangs, die in der Gesamtkonstruktion verbleiben und diese nachhaltig schwächen. Häufig kommt es zu plötzlichen Brüchen am Arbeitsplatz oder beim Einsetzen der Restauration.

Die WO 2010/057584 A1 schlägt zur Vereinfachung der Prothesenherstellung einen Fräsblock mit einer rosafarbenen Komponente und einer zahnfarbenen Komponente vor, wobei sowohl die rosafarbene Komponente als auch die zahnfarbene Komponente im Wesentlichen aus PMMA oder einem (meth-)acrylatbasierten Kunststoff besteht. Ferner wird die Verwendung eines solchen Fräsblocks zur Herstellung einer Oberkiefer-Vollprothese oder einer Unterkiefer-Vollprothese mit einem CAD/CAM-Verfahren mittels einer numerisch gesteuerten Fräsanlage beschrieben.

Auch die WO 2013/068124 beschreibt einen Fräsblock mit zwei Bereichen zur Herstellung von Dentalprothesen. Der erste Bereich weist eine entsprechend der Kieferform bearbeitbare Prothesenbasis auf, die durch Fräsen an die individuelle Schleimhaut des Patienten anzupassen ist. Der zweite Bereich weist nicht-individualisierte, vorgefertigte künstliche Zähne auf, die in einer vorbestimmten Anordnung aufgestellt sind und keiner weiteren Nachbearbeitung bedürfen. Als Material für den ersten und zweiten Bereich wird u.a. Zirkonoxidkeramik genannt. Eine Anpassung an die individuelle Patientensituation im gegenüberliegenden Kiefer ist mit einem derartigen Fräsblock jedoch nicht möglich.

Die US 2021/0128283 Al beschreibt einen zweifarbigen Rohling zur Herstellung einer Prothese mittels CAD/CAM, wobei der Rohling eine untere rosafarbene Schicht zur Imitierung der Gingiva und eine obere zahnfarbene Schicht zur Imitierung von Zähnen aufweist. Als Material für die obere und untere Schicht wird u.a. jeweils Zirkonoxidkeramik genannt. Die Grenzfläche zwischen den beiden Schichten weist eine Vielzahl von konvexen Abschnitten und konkaven Abschnitten auf, so dass die obere Oberfläche der Gingiva-Schicht eine wellenförmige Gestalt aufweist. Es wird beschrieben, dass auf diese Art und Weise der natürliche Übergang zur Gingiva besonders einfach imitiert werden könne und es möglich sei, die Anzahl der Farbtonkorrekturschritte zu verringern.

EP 3 397 193 Bl offenbart einen Rohling für dentale Prothesen, der eine erste Schicht auf Basis von Zirkonoxidkeramik und eine zweite Schicht auf Basis von Zirkonoxidkeramik aufweist, wobei die erste Schicht und die zweite Schicht sich in der Farbe unterscheiden und eine Grenzfläche bilden, wobei die Grenzfläche wellenförmig mit einander abwechselnden Wellentälern und Wellenbergen ausgebildet ist und die Scheitellinien der Wellenberge, in der Draufsicht auf die Grenzfläche betrachtet, sich strahlenförmig erstrecken, wobei der Rohling mindestens teilweise kreisbogenförmig ist. 1

US2020015944A1 offenbart einen Rohling für dentale Prothesen, der eine erste Schicht auf Basis von PMMA und eine zweite Schicht auf Basis von PMMA aufweist, wobei die erste Schicht und die zweite Schicht sich in der Farbe unterscheiden und eine Grenzfläche bilden, wobei die Grenzfläche im Zahnbogenverlauf wellenförmig mit einander abwechselnden Wellentälern und Wellenbergen ausgebildet ist und die Kammlinien der Wellenberge, in der Draufsicht auf die Grenzfläche betrachtet, sich im Bereich der frontzähne von oral nach vestibulär betrachtet strahlenförmig erstrecken, wobei der Rohling mindestens teilweise kreisbogenförmig ist und die Kammlinien von distal nach mesial ansteigend verlaufen mit einer durchschnittlichen Steigung von 15 Grad oder mehr.

Die EP 3 064 170 Al und EP 3 597 143 Al offenbaren jeweils einen Prothesenrohling, der aus einem fleischfarbenen Kunststoffmaterial und einem zahnfarbenen Kunststoffmaterial aufgebaut ist, wobei die Grenzfläche zwischen den Materialien, im Zahnbogenverlauf betrachtet, wellenförmig ausgebildet ist.

Es stellt somit eine große Herausforderung dar, Rohlinge bereitzustellen, die den vielfältigen Anforderungen für einen Einsatz im Bereich der Dentaltechnik, insbesondere zur Herstellung von Dentalprothesen, gerecht werden. Derartige Rohlinge sollen nicht nur einfach herstellbar sein, sondern sie sollen auch einfach zur gewünschten Geometrie formbar sein und trotzdem hochfeste Restaurationen ergeben. Schließlich sollen die Rohlinge zu einer Optik führen, die der des natürlichen Zahnmaterials und der natürlichen Mundschleimhaut nahekommt, damit eine aufwendige nachträgliche Erzeugung der gewünschten optischen Eigenschaften der Dentalprothesen entfallen kann.

Erfindungsgemäß sollen die oben genannten Probleme vermieden werden. Der Erfindung liegt mithin insbesondere die Aufgabe zugrunde, einen Rohling zur Verfügung zu stellen, der einfach herstellbar ist, dem maschinell in einfacher Weise die Form der gewünschten Dentalrestauration gegeben werden kann und der nach der Formgebung zu einer präzisen und hochfesten Dentalprothese umgewandelt werden kann, wobei mit dem Rohling die optische Erscheinung von natürlichem Zahnmaterial und natürlicher Mundschleimhaut sehr gut nachgeahmt werden kann.

Diese Aufgabe wird durch den Rohling nach den Ansprüchen 1 bis 18 gelöst. Gegenstand der Erfindung ist ebenfalls das Verfahren zur Herstellung des Rohlings nach den Ansprüchen 19 bis 21, die Verwendung des Rohlings nach Anspruch 22 sowie das Verfahren zur Herstellung von Dentalprothesen nach den Ansprüchen 23 bis 25.

Die Erfindung betrifft einen Rohling für dentale Prothesen, **d.h.** einen dentalen Prothesenrohling. Der erfindungsgemäße Rohling zeichnet sich dadurch aus, dass er eine erste Schicht auf Basis von Zirkonoxidkeramik und eine zweite Schicht auf Basis von Zirkonoxidkeramik aufweist, wobei die erste Schicht und die zweite Schicht sich in der Farbe unterscheiden und eine Grenzfläche bilden, wobei die Grenzfläche im Zahnbogenverlauf wellenförmig mit einander abwechselnden Wellentälern und Wellenbergen ausgebildet ist und die Scheitellinien der Wellenberge, in der Draufsicht auf die Grenzfläche betrachtet, sich in mesial-distaler Richtung strahlenförmig erstrecken, wobei der Rohling mindestens teilweise kreisbogenförmig ist und der Winkel zwischen einer fiktiven Geraden, die den tiefsten Punkt des Wellentals für den zu erstellenden Zweiten Molaren mit dem tiefsten Punkt des Wellentals für den zu erstellenden mittleren Schneidezahn verbindet, und der Projektion dieser fiktiven Geraden auf eine Grundfläche des kreisbogenförmigen Rohlings 2,0° bis 4,5° beträgt.

Der Begriff "auf Basis von" bedeutet, dass die erste und die zweite Schicht des Rohlings, bezogen auf die Masse der Summe aller Bestandteile der Schicht, überwiegend Zirkonoxid, d.h. ZrO₂, enthalten. Neben ZrO₂ und geringen Mengen an der Verunreinigung HfO₂ können die erste und/oder die zweite Schicht beispielsweise Komponenten zur Einstellung der Farbe, wie z.B. Fe, Tb, Ce, Pr, Mn, Cr, Ni, Co, Nd, Dy, Eu, Er, V und/oder Ti, und/oder Komponenten zur Einstellung der Sinterkinetik, wie z.B. Mg, Al, Y, Ce, La, Yb, Gd, Ga und/oder In, enthalten. Ferner können die erste und/oder die zweite Schicht Mischungen von Zirkonoxid mit anderen Keramiken oder Spinellen in Form eines Komposits und/oder Mischungen von Zirkonoxid mit Pigmenten enthalten.

Mit Unterschieden in der Farbe sind Unterschiede im Farbton im engeren Sinne und/oder Unterschiede in der Transluzenz, Opaleszenz oder Fluoreszenz gemeint. Der Begriff "Transluzenz" beschreibt dabei die Lichtdurchlässigkeit. Die Farbe kann insbesondere durch ihren Lab-Wert oder durch einen in der Dentalindustrie üblichen Farbschlüssel charakterisiert werden. Des Weiteren ist es nicht notwendig, dass die Unterschiede in der Farbe der ersten und zweiten Schicht im Rohling mit dem menschlichen Auge zu erkennen sind. Vielmehr kann ein Unterschied im Farbton und/oder der Transluzenz erst nach einem Sinterschritt oder einer Wärmebehandlung sichtbar werden. Gleichermaßen umfasst der Begriff "Farbgradient" neben einem Gradienten des Farbtons Gradienten der Transluzenz, Opaleszenz oder Fluoreszenz.

Die Grenzfläche zwischen erster und zweiter Schicht ist im Zahnbogenverlauf der zu erstellenden Dentalprothese wellenförmig ausgestaltet. Das heißt, dass in einer Seitenansicht auf eine bogenförmige, insbesondere parabel- oder halbkreisförmige, Schnittfläche durch den Rohling die Grenzfläche zwischen erster und zweiter Schicht wellenförmig ausgebildet ist. Weist der Rohling beispielsweise die Form einer Scheibe auf, so erstreckt sich die Schnittfläche von der oberen Grundfläche zu der unteren Grundfläche der Scheibe und verläuft im Wesentlichen parallel zur Mantelfläche der Scheibe. Die Wellenform weist abwechselnd Wellentäler und Wellenberge auf. Die Wellentäler können auch als Nuten oder Vertiefungen und die Wellenberge auch als Rippen oder Erhöhungen bezeichnet werden. Die Spitzen eines Wellenbergs und auch die - nach unten gewandten - Spitzen eines Wellentals bilden je eine Scheitellinie. Mit anderen Worten: Die Scheitel eines Wellenbergs oder eines Wellentals bilden je eine Linie, insbesondere eine gerade Linie. Die Scheitellinien der Wellenberge, und vorzugsweise auch die Scheitellinien der Wellentäler, erstrecken sich, in einer Draufsicht auf die Grenzfläche bzw. die Grundfläche des Rohlings betrachtet, von innen nach außen, d.h. von mesial nach distal. Der Begriff "wellenförmig" wird vorliegend nicht einschränkend nur zur Beschreibung von rein sinusförmigen Wellenverläufen verwendet, sondern schließt allgemein jegliche Verläufe von sich abwechselnden erhöhten und vertieften Bereichen ein.

Des Weiteren ist die Geometrie der Grenzfläche zwischen erster und zweiter Schicht des Rohlings derartig gestaltet, dass die Grenzfläche in mesial-distaler Richtung strahlenförmig ausgebildet ist. Das heißt, dass sich in einer Draufsicht auf die Grenzfläche des Rohlings die Scheitellinien der Wellenberge von einem zentralen Bereich des Rohlings in radialer Richtung, d.h. nach außen, in einer Strahlenform, vorzugsweise in Form gerader Linien, erstrecken. Die dreidimensionale Wellen- und Strahlengeometrie basiert bevorzugt auf den Daten von einer Vielzahl echter Patientenfälle.

Der erfindungsgemäße Rohling zeichnet sich somit insbesondere dadurch aus, dass mit den zwei verschiedenfarbigen Schichten sowie der integrierten Wellen- und Strahlengeometrie der Schichten ein gewünschter Farbverlauf erzeugt wird, so dass in der fertigen Dentalprothese die Farbe von Zähnen und der Gingiva sowie der Verlauf des Übergangs von Zahnmaterial zur Gingiva besonders gut imitiert werden kann. Dabei kann die Wellenform der ersten Schicht den Gingiva-Saums besonders gut abbilden. Durch die sich strahlenförmig erstreckende Wellenstruktur kann unabhängig von der Größe des erforderlichen Zahnbogens stets gleichsam automatisch der Gingiva-Saum erzeugt werden. Dies stellt ein besonderer Vorteil gegenüber der aus der US 2021/0128283 A1 bekannten schachbrettartigen Verteilung von konvexen und konkaven Bereichen dar.

Des Weiteren zeichnet sich der erfindungsgemäße Rohling dadurch aus, dass durch die Verwendung von Schichten auf Basis von Zirkonoxidkeramik hochfeste Dentalrestaurationen hergestellt werden können, die die Anforderungen an die mechanischen Eigenschaften von z.B. weitspannigen Dentalrestaurationen, wie Prothesen, vollständig erfüllen. Daneben führt die Verwendung von ZrO₂ als Werkstoff zu weiteren Vorteilen. So kann bei Zirkonoxidkeramiken im Vergleich zu Rohlingen auf Basis von Kunststoffmaterialien der Rohling insgesamt flacher ausgebildet werden, so dass die Gesamthöhe des Rohlings verringert und die Bearbeitung des Rohlings in üblichen CAM-Fräseinheit, z.B. aufgrund weniger Hinterschnitte im Design, vereinfacht werden kann.

Mit dem erfindungsgemäßen Rohling kann somit nicht nur die optische Erscheinung von natürlichem Zahnmaterial und natürlicher Mundschleimhaut sehr gut nachgeahmt werden. Dem erfindungsgemäßen Rohling lässt sich weiterhin in besonders einfache Weise die Form der gewünschten Dentalprothese geben. Dies wird überraschenderweise durch eine Kombination der besonderen Gestaltung der Grenzfläche zwischen der ersten und der zweiten Schicht und der Verwendung von Zirkonoxid als Material der ersten und der zweiten Schicht erreicht. Der erfindungsgemäße Rohling ermöglicht eine effiziente monolithische Fertigung in den Bereichen der digitalen festsitzenden und bedingt abnehmbaren Prothetik, so dass eine Teil- oder Totalprothese in einem Fräsvorgang und wenigen manuellen Arbeitsschritten gefertigt werden kann. Auch ist bei dem erfindungsgemäßen Rohling nach der Formgebung, z.B. durch CAM-Fräsung, keine nachträgliche Infiltration mit einer Färbelösung im Gingiva-Bereich notwendig, die in der Vergangenheit zu häufigen Versagensfällen der Restauration in vivo geführt hat. Dies garantiert eine spannungsfreie monolithische Fertigung und damit eine langzeitstabile Restauration.

In einer bevorzugten Ausführungsform des erfindungsgemäßen Rohlings ist die Geometrie der Grenzfläche zwischen erster und zweiter Schicht des Rohlings derartig gestaltet, dass die Grenzfläche, in der Draufsicht auf die Grenzfläche betrachtet, im Bereich der zu erstellenden Frontzähne der Prothese in mesial-distaler Richtung fächerförmig ausgebildet ist. Das heißt, dass sich in einer Draufsicht auf die Grenzfläche des Rohlings die Scheitellinien der Wellenberge von einem Mittelpunkt eines Teilabschnitts des zu erstellenden Zahnbogens in radialer Richtung, d.h. nach außen, in einer Fächerform erstrecken. Das heißt, dass sich im Bereich der zu erstellenden Frontzähne die Wellenberge, und vorzugsweise auch Wellentäler, in oral-vestibulärer Richtung betrachtet vorzugsweise fächerförmig, also von einem StrahlenMittelpunkt ausgehend radial, erstrecken.

Des Weiteren ist es bevorzugt, dass die Grenzfläche zwischen erster und zweiter Schicht im Bereich der zu erstellenden Molaren - in der Draufsicht auf die Grenzfläche betrachtet - in oral-bukkaler Richtung strahlenförmige Scheitellinien der Wellentäler und insbesondere zueinander im Wesentlichen parallele Scheitellinien der Wellentäler, und vorzugsweise auch Wellenberge, aufweist. Der Ausdruck "im Wesentlichen parallel" bedeutet, dass die Scheitellinien der Wellentäler, und vorzugsweise auch Wellenberge, sich parallel oder um höchstens 10 Grad, insbesondere höchsten 5 Grad, von der Parallelität abweichend erstrecken. Die Ausgestaltung von parallelen Scheitellinien der Wellentäler im Bereich der zu erstellenden Molaren bietet insbesondere den Vorteil einer besseren Ästhetik im Seitenzahnbereich.

Insgesamt erlauben es die beiden obigen Ausführungsformen einer fächerförmigen Ausgestaltung der Scheitellinien der Wellenberge im Frontzahnbereich und einer parallelen Ausgestaltung der Scheitellinien der Wellentäler im Seitenzahnbereich, sowohl die Funktion als auch die Ästhetik der finalen Dentalprothese zu verbessern. Insbesondere ist es möglich, sowohl für kleine als auch für große Zahnbögen geeignete Zahnsätze bereitzustellen. Bei großen Zahnbögen wird der Zahnbogen etwas weiter radial außen, also in vestibulärer Richtung verlagert, gefräst, und bei kleinen Zahnbögen wird dieser weiter innen, also nach oral verlagert, gefräst. Durch die erfindungsgemäße Parallelisierung der Wellenberge und Wellentäler der Grenzfläche für den Bereich der Molaren steht auch bei kleinen Zahnbögen erfindungsgemäß eine vergleichsweise große Kaufläche zur Verfügung.

Mithin kann der erfindungsgemäße Rohling in einer bevorzugten Ausgestaltung im Bereich der zu erstellenden mittleren Schneidezähne fächerförmige Wellentäler und Wellenberge mit einem echten Strahlmittelpunkt aufweisen. Dieser wird von der Kammlinie des Wellenbergs für den zu erstellenden Eckzahn jedoch nicht mehr richtig getroffen. Vielmehr ist diese Kammlinie weniger fächerförmig und paralleler zur benachbarten Kammlinie des seitlichen Schneidezahns. Dies gilt auch für die Erste Prämolaren-Kammlinie, die wiederum einen Verlauf hat, der der Parallelität noch stärker angenähert ist. Die nächstfolgenden Kammlinien, also die Wellenberg-Kammlinien für die Zweiten Prämolaren, die Ersten Molaren und die Zweiten Molaren, sind zueinander vollständig parallel.

Ferner verlaufen die Kammlinien von distal nach mesial vorzugsweise ansteigend. Die Grenzfläche ist also vorzugsweise schräg in dem Rohling angeordnet. Dadurch kann der Farb- und Transluzenzverlauf von natürlichen Zähnen, insbesondere Frontzähnen, besonders gut nachgeahmt werden.

In einer weiteren bevorzugten Ausführungsform weist die zweite Schicht des erfindungsgemäßen Rohlings einen kontinuierlichen, d.h. linearen, oder diskontinuierlichen, d.h. nicht linearen, Farbgradienten auf. Auch dadurch kann der Farb- und Transluzenzverlauf von natürlichen Zähnen, insbesondere Frontzähnen, besonders gut nachgeahmt werden.

Besonders bevorzugt enthält die Zirkonoxidkeramik der zweiten Schicht Yttrium und der Farbgradient, insbesondere ein Transluzenzverlauf, wird vorzugsweise durch einen Gradienten der Menge an Yttrium, d.h. durch eine graduell sich verändernde Menge an Yttrium, ausgebildet. Mithin weist der Rohling vorzugsweise einen kontinuierlichen, d.h. linearen, oder diskontinuierlichen, d.h. nicht linearen, Gradienten der Menge an Yttrium auf. Insbesondere steigt der Gehalt an Yttrium von der Grenzfläche zwischen erster und zweiter Schicht zu der der Grenzfläche gegenüberliegenden äußeren Oberfläche der zweiten Schicht hin an. Dieser Anstieg des Yttriumgehalts kann stufenlos oder stufenweise erfolgen.

In einer Ausführungsform weist die zweite Schicht des erfindungsgemäßen Rohlings mindestens zwei diskrete Schichten auf, die sich in ihrem Yttriumgehalt unterscheiden.

Vorzugsweise weist die zweite Schicht eine innere Schicht und eine äußere Schicht auf, wobei die innere Schicht an die Grenzfläche zwischen erster und zweiter Schicht angrenzt und die äußere Schicht an die der Grenzfläche gegenüberliegenden äußeren Oberfläche der zweiten Schicht angrenzt. Die innere Schicht dient insbesondere als sog. Dentinschicht, d.h. zur Nachahmung des Dentinbereichs von natürlichen Zähnen. Die äußere Schicht dient insbesondere als Schneideschicht, d.h. zur Nachahmung einer Schmelzschicht von natürlichen Zähnen.

In dieser Ausführungsform ist es besonders bevorzugt, dass
(a) die innere Schicht einen Yttrium-Gehalt von 2,0 bis 6,0 Mol-%, insbesondere 3,0 bis 5,0 Mol-%, aufweist und
(c) die äußere Schicht einen Yttrium-Gehalt von 3,5 bis 8,0 Mol-%, insbesondere 4,0 bis 7,5 Mol-%, aufweist,
wobei der Yttrium-Gehalt als Anteil der Stoffmenge von Y₂O₃ bezogen auf die Summe der Stoffmengen von Y₂O₃, ZrO₂ und HfO₂ definiert ist.

Optional befindet sich zwischen der inneren Schicht und der äußeren Schicht eine sog. Zwischenschicht. Bei Vorhandensein einer Zwischenschicht ist es besonders bevorzugt, dass
(a) die innere Schicht einen Yttrium-Gehalt von 2,0 bis 6,0 Mol-%, insbesondere 3,0 bis 5,0 Mol-%, aufweist,
(b) die Zwischenschicht einen Yttrium-Gehalt von 3,0 bis 7,0 Mol-%, insbesondere 3,5 bis 4,5 Mol-%, aufweist, und
(c) die äußere Schicht einen Yttrium-Gehalt von 3,5 bis 8,0 Mol-%, insbesondere 4,0 bis 7,5 Mol-%, aufweist,
wobei der Yttrium-Gehalt als Anteil der Stoffmenge von Y₂O₃ bezogen auf die Summe der Stoffmengen von Y₂O₃, ZrO₂ und HfO₂ definiert ist.

Des Weiteren ist es bevorzugt, dass die innere Schicht, die äußere Schicht und optional die Zwischenschicht zusätzlich zu Y₂O₃ Oxide zur Einstellung der Farbe, insbesondere Oxide von Fe, Cr, Mn, Tb, Pr, Ce, Ni, Co, Nd, Dy, Eu, Er, V und/oder Ti, und Oxide zur Einstellung der Sinterkinetik, insbesondere Mg, La, Al, Ce, Yb, Gd, Ga und/oder In, enthalten.

Vorzugsweise enthalten die innere Schicht, die äußere Schicht und optional die Zwischenschicht zusätzlich zu Y₂O₃ zur Einstellung der Farbe mindestens ein Oxid von Fe, Cr, Mn, Tb und Pr und besonders bevorzugt Oxide von sämtlichen dieser Elemente. Vorzugsweise enthalten die innere Schicht, die äußere Schicht und optional die Zwischenschicht zusätzlich zu Y₂O₃ weiterhin zur Einstellung der Sinterkinetik mindestens ein Oxid von Mg, La, Y und Al und besonders bevorzugt Oxide von sämtlichen dieser Elemente.

Die zweite Schicht kann vorzugsweise mindestens eine und insbesondere alle der folgenden Komponenten in den angegebenen Mengen enthalten:

| | |
|---|---|
| Komponente ZrO₂ + HfO₂ Y₂O₃ Fe₂O₃ Cr₂O₃ Mn₂O₃ Pr₂O₃ | Gew.-% 82.5 - 96.5, insbesondere 84.5 - 95.0 3.5 - 11.0, insbesondere 5.0 - 9.0 0 - 0.15, insbesondere 0.005 - 0.12 0 - 0.025, insbesondere 0.0002 - 0.01 0 - 0.002, insbesondere 0.00005 - 0.0012 0 - 0.02, insbesondere 0.0001 - 0.015 |
| Tb₂O₃ | 0 - 0.02, insbesondere 0.00005 - 0.015 |
| Er₂O₃ | 0 - 1.0, insbesondere 0.01 - 0.65 |
| MgO | 0 - 0.05, insbesondere 0 - 0.025 |
| Al₂O₃ | 0 - 0.25, insbesondere 0 - 0.15 |
| La₂O₃ | 0 - 1.0, insbesondere 0 - 0.55 |

Weiterhin kann die innere Schicht vorzugsweise mindestens eine und insbesondere alle der folgenden Komponenten in den angegebenen Mengen enthalten:

| Komponente | Gew.-% |
|---|---|
| ZrO₂ + HfO₂ | 85.0 - 96.5, insbesondere 86.5 - 95.5 |
| Y₂O₃ | 3.5 - 8.5, insbesondere 4.5 - 7.0 |
| Fe₂O₃ | 0 - 0.15, insbesondere 0.005 - 0.12 |
| Cr₂O₃ | 0 - 0.025, insbesondere 0.0002 - 0.01 |
| Mn₂O₃ | 0 - 0.002, insbesondere 0.00005 - 0.0012 |
| Pr₂O₃ | 0 - 0.02, insbesondere 0.0001 - 0.015 |
| Tb₂O₃ | 0 - 0.02, insbesondere 0.00005 - 0.015 |
| Er₂O₃ | 0 - 1.0, insbesondere 0.01 - 0.65 |
| MgO | 0 - 0.025, insbesondere 0 - 0.005 |
| Al₂O₃ | 0 - 0.25, insbesondere 0 - 0.15 |
| La₂O₃ | 0 - 1.0, insbesondere 0 - 0.55 |

Weiterhin kann die äußere Schicht vorzugsweise mindestens eine und insbesondere alle der folgenden Komponenten in den angegebenen Mengen enthalten:

| Komponente | Gew.-% |
|---|---|
| ZrO₂ + HfO₂ | 81.5 - 95.0, insbesondere 82.5 - 93.5 |
| Y₂O₃ | 5.0 - 12.0, insbesondere 6.5 - 11.0 |
| Fe₂O₃ | 0 - 0.1, insbesondere 0.0035 - 0.085 |
| Cr₂O₃ | 0 - 0.025, insbesondere 0.0002 - 0.01 |
| Mn₂O₃ | 0 - 0.002, insbesondere 0.00005 - 0.0012 |
| Pr₂O₃ | 0 - 0.02, insbesondere 0.0001 - 0.015 |
| Tb₂O₃ | 0 - 0.02, insbesondere 0.00005 - 0.015 |
| Er₂O₃ | 0 - 0.6, insbesondere 0.01 - 0.5 |
| MgO | 0 - 0.025, insbesondere 0 - 0.005 |
| Al₂O₃ | 0 - 0.25, insbesondere 0 - 0.15 |
| La₂O₃ | 0 - 0.55, insbesondere 0 - 0.15 |

Weiterhin kann die Zwischenschicht vorzugsweise mindestens eine und insbesondere alle der folgenden Komponenten in den angegebenen Mengen enthalten:

| Komponente | Gew.-% |
|---|---|
| ZrO₂ + HfO₂ | 83.0 - 96.0, insbesondere 84.0 - 94.5 |
| Y₂O₃ | 4.0 - 11.0, insbesondere 5.0 - 9.0 |
| Fe₂O₃ | 0 - 0.12, insbesondere 0.005 - 0.1 |
| Cr₂O₃ | 0 - 0.025, insbesondere 0.0002 - 0.01 |
| Mn₂O₃ | 0 - 0.002, insbesondere 0.00005 - 0.0012 |
| Pr₂O₃ | 0 - 0.02, insbesondere 0.0001 - 0.015 |
| Tb₂O₃ | 0 - 0.02, insbesondere 0.00005 - 0.015 |
| Er₂O₃ | 0 - 1.0, insbesondere 0.01 - 0.65 |
| MgO | 0 - 0.02, insbesondere 0 - 0.005 |
| Al₂O₃ | 0 - 0.25, insbesondere 0 - 0.15 |
| La₂O₃ | 0 - 1.0, insbesondere 0 - 0.55 |

Die Zirkonoxidkeramik der ersten Schicht enthält Zirkonoxid, das vorzugsweise mit Erbium und/oder Yttrium stabilisiert ist. Vorzugsweise weist die Zirkonoxidkeramik der ersten Schicht einen Erbium-Gehalt von 0,0 bis 4,5 Mol-%, insbesondere 0,5 bis 4,25 Mol-%, besonders bevorzugt 1,5 bis 3,5 Mol.-%, auf, wobei der Erbium-Gehalt als Anteil der Stoffmenge von Er₂O₃ bezogen auf die Summe der Stoffmengen von Er₂O₃, ZrO₂ und HfO₂ definiert ist. Des Weiteren weist die Zirkonoxidkeramik der ersten Schicht vorzugsweise einen Yttrium-Gehalt von 0,0 bis 4,5 Mol-%, insbesondere

0,5 bis 4,25 Mol-%, besonders bevorzugt 0,75 bis 2,0 Mol.-%, auf, wobei der Yttrium-Gehalt als Anteil der Stoffmenge von Y₂O₃ bezogen auf die Summe der Stoffmengen von Y₂O₃, ZrO₂ und HfO₂ definiert ist. Des Weiteren ist es bevorzugt, dass die Summe des Erbium- und Yttrium-Gehalts 1,5 bis 6,0 Mol.%, insbesondere 2,0 bis 4,5 Mol.%, besonders bevorzugt 2,5 bis 4,0 Mol-% beträgt. Da die erste Schicht des Rohlings zur Bildung des Gingiva-Bereichs der zu erstellenden Dentalrestauration dient, ist eine Ausbildung eines Farbgradienten oder Stoffgradienten von Yttrium in der ersten Schicht nicht vorteilhaft. Somit ist der Yttrium-Gehalt innerhalb der ersten Schicht vorzugsweise im Wesentlichen konstant. Alternativ kann die erste Schicht einen Gradienten aufweisen, der eine feste und bewegliche Gingiva repräsentieren soll.

Die Zirkonoxidkeramik der ersten Schicht enthält vorzugsweise mindestens eine und insbesondere alle der folgenden Komponenten in den angegebenen Mengen:

| Komponente | Gew.-% |
|---|---|
| ZrO₂ + HfO₂ | 86,0 - 97,0, vorzugsweise 90,0 - 94,0 |
| Er₂O₃ | 0,5 - 10,0, |
| | vorzugsweise 4,0 - 8,0 |
| Y₂O₃ | 0,5 - 8,0, |
| | vorzugsweise 1,0 - 4,0 |
| erstes farbgebendes Oxid | 0,0 - 0,5, |
| | vorzugsweise 0,0 - 0,25, |
| | besonders bevorzugt 0,0 - 0,1 |
| zweites farbgebendes Oxid | 0,0 - 0,1, |
| | vorzugsweise 0,0 - 0,05, |
| | besonders bevorzugt 0,0 - 0,025 |

wobei das erste farbgebende Oxid ausgewählt ist aus der Gruppe bestehend aus Fe₂O₃, Tb₂O₃, Pr₂O₃ und V₂O₅ und insbesondere eine zusätzliche Gelbfärbung der Zirkonoxidkeramik bewirken kann, und wobei das zweite farbgebende Oxid ausgewählt ist aus der Gruppe bestehend aus Mn₂O₃ , Cr₂O₃ und CoO und insbesondere eine zusätzliche Graufärbung der Zirkonoxidkeramik bewirken kann.

In einer Ausführungsform ist es bevorzugt, dass die erste Schicht des Rohlings Zirkonoxidkeramik oder eine Mischung von Zirkonoxidkeramik mit einem oder mehreren Materialien ausgewählt aus der Gruppe bestehend aus durch Aluminiumoxid verstärktes Zirkonoxid (ATZ, alumina toughened zircona), durch Zirkonoxid verstärktes Aluminiumoxid (ZTA, zircona toughened alumina), Spinellen, Pigmenten oder Mischungen davon enthält.

Durch Aluminiumoxid verstärktes Zirkonoxid (ATZ) enthält 5 bis 40 Gew.-%, insbesondere etwa 20 Gew.-%, Al₂O₃ und 60 bis 95 Gew.- %, insbesondere etwa 80 Gew.-%, ZrO₂. Der Al₂O₃-Anteil kann mit MgO, wie z.B. 50 Gew.-ppm bis 3 Gew.-%, dotiert sein. Der ZrO₂-Anteil kann mit Y₂O₃, wie z.B. 1 bis 3 Mol-%, dotiert sein.

Durch Zirkonoxid verstärktes Aluminiumoxid (ZTA) enthält 60 bis 95 Gew.-%, insbesondere etwa 80 bis 90 Gew.-%, Al₂O₃ und 5 bis 40 Gew.-%, insbesondere etwa 10 bis 20 Gew.-%, ZrO₂. Der Al₂O₃-Anteil kann mit MgO, wie z.B. 50 Gew.-ppm bis 3 Gew.-%, dotiert sein. Der ZrO₂-Anteil kann mit Y₂O₃, wie z.B. 1 bis 3 Mol-%, dotiert sein.

Bei Mischungen der Zirkonoxidkeramik mit ATZ und ZTA können im Endgefüge mittels REM diskrete Al₂O₃- und ZrO₂-Kristallite nachgewiesen werden. Im Gegensatz dazu sind in aktuellen Zirkonoxidkeramiken für die dentale Anwendung im Gefüge in der Regel keine Al₂O₃-Kristallite mittels REM nachweisbar. Dies liegt vermutlich an der geringeren Menge an Al₂O₃, üblicherweise etwa 0,05 bis 0,1 Gew.-%, und seiner homogenen Verteilung. ATZ- und ZTA-Komposite können die mechanischen Eigenschaften des Rohlings positiv beeinflussen, insbesondere sowohl die Biaxialfestigkeit als auch die Bruchzähigkeit erhöhen. Eine Erhöhung der Bruchzähigkeit wird insbesondere dann erreicht, wenn der ZrO₂-Anteil von ATZ oder ZTA durch 1.5 bis 2.5 Mol.% Y₂O₃ stabilisiert ist.

Als Spinelle eignen sich insbesondere Spinelle ausgewählt aus der Gruppe bestehend aus MgAl₂O₄, SrAl₂O₄, La-Spinell, Y-Spinell und Mischungen daraus, wie z.B. LaMg-Spinelle. Spinelle können die mechanischen Eigenschaften des Rohlings positiv beeinflussen, insbesondere sowohl die Biaxialfestigkeit als auch die Bruchzähigkeit erhöhen. Eine Erhöhung der Bruchzähigkeit wird insbesondere dann erreicht, wenn der ZrO₂-Anteil der Zirkonoxidkeramik durch 1.5 bis 2.5 Mol.% Y₂O₃ stabilisiert ist. In dem Komposit aus Zirkonoxidkeramik und Spinell wächst der Spinell insbesondere bei hohen Sintertemperaturen meist stäbchenförmig, wodurch die Bruchzähigkeit des Komposits positiv beeinflusst wird.

Während die zweite Schicht des erfindungsgemäßen Rohlings vorzugsweise zahnfarben ist und auch nach einer Wärmebehandlung des Rohlings einen zahnfarbenen Farbton behält, kann die erste Schicht weißlich oder rosafarben ausgestaltet sein und einen solchen Farbton auch nach einer Wärmebehandlung behalten. Im Falle einer ersten Schicht mit weißlichem Farbton ist es üblich nach der Formgebung und einer Wärmebehandlung den Gingiva-Bereich nachträglich zu beschichten, um die Ästhetik des Gingiva-Bereichs in der fertigen Dentalrestauration möglichst optimal zu imitieren. Ein derartiger Schritt kann im Fall einer ersten Schicht mit einer voreingefärbten ersten Schicht entfallen, so dass in dieser Ausführungsform eine einfachere Herstellung von z.B. Dentalprothesen möglich ist.

Besonders vorteilhaft sind erfindungsgemäße Rohlinge, bei denen die Zirkonoxidkeramik der ersten Schicht und die Zirkonoxidkeramik der zweiten Schicht vorgesintert sind. Dadurch wird die Verarbeitbarkeit und Präzision bei einer späteren maschinellen Bearbeitung zur Herstellung von Dentalrestaurationen verbessert. Insbesondere wird aufgrund der geringeren Festigkeit von Zirkonoxid im vorgesinterten Zustand eine einfache, zeitsparende und Fräswerkzeug-schonende Formgebung der Rohlinge ermöglicht. Vorzugsweise weisen die Zirkonoxidkeramik der ersten Schicht und die Zirkonoxidkeramik der zweiten Schicht eine Dichte von 1,8 bis 4,4 g/cm³, insbesondere 2,5 bis 4,0 g/cm³, auf.

Bei derartigen vorgesinterten Rohlingen ist es für die Herstellung von Dentalrestaurationen notwendig, die Rohlinge einem Sinterschritt, **d.h.** einer Wärmebehandlung, zu unterziehen, damit die gewünschten mechanischen Eigenschaften, insbesondere eine hohe Festigkeit und Härte, erreicht werden. Bei einer solchen Wärmebehandlung kommt es zu einem Sinterschrumpf der Zirkonoxidkeramik. Es ist somit bei der Verwendung von vorgesinterten Rohlingen notwendig, dass die Wellen- und Strahlengeometrie der erfindungsgemäßen Rohlinge im Vergleich zu Rohlingen auf Basis von Materialien ohne Sinterschrumpf, **z.B.** Kunststoffmaterialien, vergrößert ist. Vorzugsweise sind bei einem erfindungsgemäßen Rohling im vorgesinterten Zustand die Abmessungen der Geometrie um einen Faktor von 1,200 bis 1,250, insbesondere von 1,22 bis 1,25, vergrößert. Bei einem erfindungsgemäßen Rohling im Grünzustand sind die Abmessungen der Geometrie um einen Faktor von 1,250 bis 1,350, insbesondere etwa 1,275 vergrößert.

Der erfindungsgemäße Rohling weist vorzugsweise eine biaxiale Bruchfestigkeit von 10 bis 150 MPa, insbesondere 20 bis 120 MPa, besonders bevorzugt 25 bis 80 MPa, auf. Die biaxiale Bruchfestigkeit wurde gemäß ISO 8672 (2008) (Kolben-auf-drei-Kugeln-Prüfung) bestimmt.

Ferner weist der erfindungsgemäße Rohling vorzugsweise eine Vickers-Härte Hv_{2.5} von 50 bis 1000 MPa, insbesondere 300 bis 850 MPa, besonders bevorzugt 300 bis 700 MPa, auf. Die Vickers-Härte wurde nach ISO 14705:2016 bei einer Last von 2,5 kg gemessen.

Des Weiteren ist es bevorzugt, dass bei dem erfindungsgemäßen Rohling die erste Schicht und die zweite Schicht durch einstückige Herstellung miteinander verbunden sind. Dies ermöglicht eine effiziente monolithische Fertigung, **d.h.** aus einem Rohling kann eine patientenindividuelle Totalprothese in einem Fräsvorgang gefertigt werden.

Die Form des erfindungsgemäßen Rohlings ist zumindest teilweise kreisbogenförmig. Insbesondere hat der Rohling die Form einer Scheibe, besonders bevorzugt die Form einer kreisrunden Scheibe.

Weiterhin ist es bevorzugt, dass der kreisbogenförmige Rohling am Außenumfang einen Vorsprung, insbesondere einen nach außen vorspringenden Einspannrand, aufweist. Dieser Einspannrand dient als Halter in Schleif- und Fräsvorrichtungen, wie üblichen CAD/CAM-Vorrichtungen. Der Vorsprung kann aus dem gleichen Material wie der Rohling sein. In diesem Fall kann der Rohling zunächst mit einem größeren Umfang bereitgestellt werden und der Vorsprung kann durch Abdrehverfahren, **z.B.** auf einer Fräsmachine, erhältlich sein. Alternativ kann der Vorsprung aus einem unterschiedlichen Material gebildet sein und zum Beispiel durch einen Kunststoffring gebildet werden, der nachträglich auf den Rohling aufgebracht wird.

Es ist ferner bevorzugt, dass der erfindungsgemäße Rohling am Außenumfang mindestens zwei Markierungen, Nuten und/oder Abflachungen aufweist, die asymmetrisch und nicht rotationssymmetrisch zueinander angeordnet sind, also nicht diametral einander gegenüber liegen, und als Rotationsschutz bzw. Verdrehsicherung dienen. Die Lage der Wellengeometrie im Verhältnis zur Scheibe muss in alle Raumrichtungen bekannt und fixiert sein, damit die Restauration im Verhältnis zur Welle im späteren Schritt zur maschinellen Bearbeitung platziert werden kann.

Der erfindungsgemäße Rohling auf Basis von Zirkonoxidkeramik wird insbesondere für festsitzende Prothetik verwendet. Das heißt, dass der erfindungsgemäße Rohling sich insbesondere zur Herstellung von festsitzenden Dentalprothesen eignet, die durch Verschraubung oder Verklebung an Implantaten oder Restzahnanteilen, wie **z.B.** einem Zahnstumpf, befestigt werden. Dadurch kann bei erfindungsgemäßen Rohlingen zur Herstellung einer Oberkieferprothese eine Umschlagfalte und Gaumenplatte, die für die Haftung der Prothese am Gaumen des Patienten notwendig ist, wegfallen. Dies wiederum führt dazu, dass die Höhe der ersten Schicht des Rohlings, die zur Nachahmung des Gingiva-Bereichs dient, verringert werden kann. Des Weiteren ist es durch den Wegfall einer Gaumenplatte nicht notwendig, die gesamte erste Schicht rosafarben zu gestalten oder anderweitig zu färben. Vielmehr kann in Draufsicht auf die kreisbogenförmige Grundfläche des Rohlings ein innerer Bereich wegfallen oder ungefärbt bleiben.

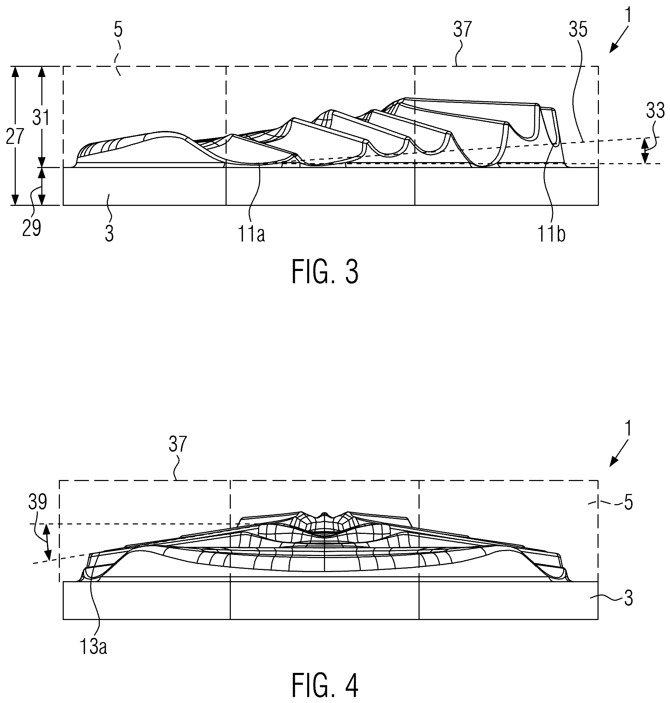

Mithin kann bei erfindungsgemäßen Rohlingen seine Höhe verringert werden, so dass sie deutlich unterhalb von 30 mm bleibt, was eine kritische Grenze für die Bearbeitung eines Rohlings in einer handelsüblichen dentalen Fräsmaschine ist. In einer bevorzugten Ausführungsform beträgt die Höhe des Rohlings, d.h. der Abstand zwischen der kreisbogenförmigen Oberseite des Rohlings und der gegenüberliegenden kreisbogenförmigen Unterseite des Rohlings, nicht mehr als 30 mm, vorzugsweise 20 bis 30 mm, besonders bevorzugt 25 bis 29 mm. Außerdem beträgt die Höhe der ersten Schicht vorzugsweise 5,0 bis 9,0 mm, insbesondere 6,0 bis 7,5 mm, besonders bevorzugt etwa 7,0 mm, und/oder die Höhe der zweiten Schicht vorzugsweise 15,0 bis 25,0 mm, insbesondere 18,0 bis 22,0 mm, besonders bevorzugt etwa 20 mm. Als Höhe der ersten Schicht wird dabei der geringste Abstand zwischen der der Grenzfläche gegenüberliegenden äußeren Oberfläche der ersten Schicht und dem tiefsten Wellental der ersten Schicht in einer Seitenansicht auf die Mantelfläche des vorzugsweise scheibenförmigen Rohlings bezeichnet. Als Höhe der zweiten Schicht wird dabei der geringste Abstand zwischen der der Grenzfläche gegenüberliegenden äußeren Oberfläche der zweiten Schicht und dem höchsten Wellenberg der zweiten Schicht in einer Seitenansicht auf die Mantelfläche des vorzugsweise scheibenförmigen Rohlings bezeichnet, wie dies weiter unten als Abstand 31 in Figur 3 illustriert wird.

Das Verhältnis der Höhe der ersten Schicht zur Höhe der zweiten Schicht beträgt vorzugsweise 1:1 bis 1:5, insbesondere 1:2,5 bis 1:3,5.

Durch die erfindungsgemäße Kombination der speziellen Wellengeometrie des Rohlings und des zu verwendenden Zirkonoxidmaterials können neben einer Verringerung der Gesamthöhe des Rohlings weitere Vorteile erreicht werden. Beispielsweise wurde überraschenderweise gefunden, dass es möglich ist, im Vergleich zu aus dem Stand der Technik bekannten Prothesenrohlingen (i) den Winkel zwischen der Grenzfläche zwischen erster und zweiter Schicht und der Oberfläche des Rohlings und/oder (ii) den Winkel zwischen der Kammlinie der Wellenberge der Grenzfläche und der Oberfläche des Rohlings zu verringern.

Insbesondere zur Eignung zur Herstellung einer Prothese im Oberkiefer ist der Rohling dadurch gekennzeichnet, dass der Winkel zwischen einer fiktiven Geraden, die den tiefsten Punkt des Wellentals für den zu erstellenden Zweiten Molaren mit den tiefsten Punkt des Wellentals für den zu erstellenden mittleren Schneidezahn verbindet, und der Projektion dieser fiktiven Gerade auf die kreisbogenförmige Grundfläche des Rohlings 2,0° bis 4,5°, vorzugsweise 2,0° bis 4,0°, besonders bevorzugt 2,5° bis 3,5° und am meisten bevorzugt etwa 3,0° beträgt. Durch einen derartigen geringen Winkel können insbesondere bei Rohlingen mit einem Farbgradienten in der zweiten Schicht die Vorteile dieses Farbgradienten besser ausgenutzt werden und der Farb- und Transluzenzverlauf von Front- und Backenzähnen der zu erstellenden Prothese kann verbessert werden.

In einer weiteren bevorzugten Ausführungsform ist der Rohling dadurch gekennzeichnet, dass der Winkel zwischen der kreisbogenförmigen Grundfläche des Rohlings und der Kammlinie der Wellenberge der wellenförmigen Grenzfläche 7° bis 13°, vorzugsweise 9° bis 11°, und besonders bevorzugt etwa 10°, beträgt.

Insbesondere in Kombination mit einer Verringerung der Gesamthöhe des Rohlings führen die Verringerung des Winkels der Grenzfläche zu der Oberfläche des Rohlings und/oder die Verringerung des Winkels der Kammlinien der Wellenberge zu der Oberfläche des Rohlings zu einer einfacheren Bearbeitbarkeit des Rohlings in einer Fräsmaschine, da in diesen Ausführungsformen weniger Hinterschnitte notwendig sind.

Die Erfindung betrifft ebenfalls ein Verfahren zur Herstellung der erfindungsgemäßen Rohlinge.

Das Verfahren zur Herstellung eines erfindungsgemäßen Rohlings zeichnet sich dadurch aus, dass
(i) ein erster und ein zweiter Grünkörper bereitgestellt werden, wobei der erste Grünkörper zur Bildung der ersten Schicht des Rohlings und der zweite Grünkörper zur Bildung der zweiten Schicht des Rohlings vorgesehen sind, und wobei eine erste Seite des ersten Grünkörpers die aus Wellental und Wellenberg gebildete Wellengeometrie der ersten Schicht aufweist und eine erste Seite des zweiten Grünkörpers die entsprechende inverse Wellengeometrie aufweist;
(ii) der erste und zweite Grünkörper übereinandergelegt werden, so dass die erste Seite des ersten Grünkörpers und die erste Seite des zweiten Grünkörpers vollflächig in engen Kontakt miteinander kommen;
(iii) der erste und zweite Grünkörper zusammengepresst werden; und
(iv) mindestens eine Wärmebehandlung durchgeführt wird, um das Zirkonoxid in vorgesintertes Zirkonoxid umzuwandeln.

Besonders bevorzugt werden in Schritt (i) separat ein erster Grünkörper und ein zweiter Grünkörper bereitgestellt, indem die Zirkonoxidkeramik-Startmaterialien für die erste Schicht und die zweite Schicht jeweils separat in eine Form gefüllt werden und die Massen anschließend jeweils uniaxial und/oder isostatisch verpresst werden. Der Druck bei diesem Pressen beträgt vorzugsweise weniger als 100 MPa.

Im Anschluss daran kann die gewünschte Formung der Oberfläche des ersten und zweiten Grünkörpers vorzugsweise durch Fräsen erreicht werden.

Des Weiteren ist es bevorzugt, dass der erste und der zweite Grünkörper in Schritt (iii) isostatisch, insbesondere bei einem Druck von mehr als 100 MPa, vorzugsweise bei einem Druck von 150 bis 1000 MPa, bevorzugt 150 bis 500 MPa, zusammengepresst werden.

Ferner ist es bevorzugt, dass in Schritt (iv) die Wärmebehandlung bei einer Temperatur von 700 bis 1200°C und vorzugsweise bei einer Temperatur von 800 bis 1100°C stattfindet. Die Dauer der Wärmebehandlung in Schritt (iv) kann vorzugsweise 5 bis 600 min, insbesondere 10 bis 300 min, in einem Temperaturbereich von 700 bis 1200°C und vorzugsweise von 800 bis 1100°C betragen.

Aufgrund der geschilderten besonderen Eigenschaften der erfindungsgemäßen Rohlinge eignen sich diese besonders zur Erzeugung von Dentalrestaurationen, insbesondere Dentalprothesen. Die Erfindung betrifft daher auch die Verwendung der erfindungsgemäßen Rohlinge zur Herstellung einer Dentalrestauration und insbesondere einer Dentalprothese, wobei die Dentalprothese vorzugsweise ausgewählt ist aus der Gruppe bestehend aus Oberkiefer-Totalprothesen, Unterkiefer-Totalprothesen, Oberkiefer-Teilprothesen und Unterkiefer-Teilprothesen. Insbesondere kann es sich bei den genannten Dentalprothesen vorteilhaft um eine Implantatrestauration handeln. Dabei können die Schnittstellen der Dentalprothese zu den Implantaten direkt gefertigt werden, so dass die Dentalprothese direkt mit dem Implantat verbunden werden kann, z.B. durch Verschraubung. Alternativ kann die Dentalprothese mit einer Basis, z.B. einer Basis aus Titan, verbunden werden, z.B. durch Verkleben, welche dann durch Verschraubung mit dem Implantat verbunden wird.

In einem weiteren Aspekt betrifft die Erfindung auch ein Verfahren zur Herstellung einer Dentalrestauration, bei dem
(i-1) einem erfindungsgemäßen Rohling durch maschinelle Bearbeitung die Form der Dentalrestauration gegeben wird,
(i-2) mindestens eine Wärmebehandlung durchgeführt wird, um das Zirkonoxid der ersten und zweiten Schicht in dichtgesintertes Zirkonoxid umzuwandeln, und
(i-3) gegebenenfalls die Oberfläche der erhaltenen Dentalrestauration endbehandelt wird.

Aus den erfindungsgemäßen Rohlingen können in einfacher Weise durch maschinelle Bearbeitung die gewünscht geformten Dentalrestaurationen, insbesondere Dentalprothesen, herausgearbeitet werden.

Die maschinelle Bearbeitung in Schritt (i-1) erfolgt üblicherweise durch materialabtragende Verfahren und insbesondere durch Fräsen und/oder Schleifen. Es ist bevorzugt, dass die maschinelle Bearbeitung mit computergesteuerten Fräs- und/oder Schleifvorrichtungen erfolgt. Besonders bevorzugt erfolgt die maschinelle Bearbeitung im Rahmen eines CAD/CAM-Verfahrens.

In Schritt (i-2) wird der Rohling einer Wärmebehandlung unterzogen, um die Bildung von dichtgesinterter Zirkonoxidkeramik herbeizuführen. Die Wärmebehandlung findet insbesondere bei einer Temperatur von 1050 bis 1700°C und bevorzugt 1100 bis 1600°C statt. Die Wärmebehandlung wird insbesondere für eine Dauer von 0 bis 240 min, bevorzugt 5 bis 180 min, besonders bevorzugt 30 bis 120 min, durchgeführt, wobei sich der Begriff "Dauer" auf die Haltezeit der Maximaltemperatur bezieht.

Die erfindungsgemäß hergestellten Dentalrestaurationen sind insbesondere Dentalprothesen und besonders bevorzugt ausgewählt aus der Gruppe bestehend aus Oberkiefer-Totalprothesen, Unterkiefer-Totalprothesen, Oberkiefer-Teilprothesen und Unterkiefer-Teilprothesen.

Die erfindungsgemäß hergestellten Dentalprothesen zeichnen sich neben hervorragenden optischen Eigenschaften durch eine besonders hohe Festigkeit aus. Vorzugsweise weist die Dentalprothese im Gingiva-Bereich, d.h. in der ersten Schicht, eine biaxiale Bruchfestigkeit gemäß ISO 6872 (2208) (Kolben-auf-drei-Kugeln-Prüfung) von mehr als 800 MPa, insbesondere mehr als 900 MPa und besonders bevorzugt mehr als 1000 MPa auf.

Des Weiteren weist die Dentalprothese im Dentin-Bereich, d.h. in der inneren Schicht der zweiten Schicht, vorzugsweise eine biaxiale Bruchfestigkeit gemäß ISO 6872 (2208) (Kolben-auf-drei-Kugeln-Prüfung) von mehr als 600 MPa, insbesondere mehr als 600 MPa und besonders bevorzugt mehr als 800 MPa auf.

Zudem weist die Dentalprothese im Schneide-Bereich, d.h. in der äußeren Schicht der zweiten Schicht, vorzugsweise eine biaxiale Bruchfestigkeit gemäß ISO 6872 (2208) (Kolben-auf-drei-Kugeln-Prüfung) von mehr als 300 MPa, insbesondere mehr als 400 MPa und besonders bevorzugt mehr als 500 MPa auf.

In dem optionalen Schritt (i-3) kann die Oberfläche der Dentalrestauration noch endbehandelt werden. Dabei ist es insbesondere möglich, bei einer Dentalprothese mit weißlichem Ginigiva-Bereich, d.h. bei Verwendung eines Rohlings mit weißlicher erster Schicht, den Gingiva-Bereich mit Schicht-, Verblend-, Mal- oder Charakterisierungsmassen zu überdecken, um den Farbton natürlicher Mundschleimhaut besonders gut nachzuahmen. Alternativ oder zusätzlich kann in Schritt (i-3) eine Oberflächenpolitur der Dentalrestauration durchgeführt werden.

Weitere Merkmale und Vorteile ergeben sich aus der nachfolgenden Beschreibung von Ausführungsbeispielen der vorliegenden Erfindung anhand der Zeichnungen. Es zeigt
- Fig. 1: eine schematische perspektivische Ansicht eines erfindungsgemäßen Rohlings;
- Fig. 2: eine schematische Draufsicht auf die erste Schicht des in Figur 1 dargestellten Rohlings;
- Fig. 3: eine schematische Seitenansicht eines erfindungsgemäßen Rohlings; und
- Fig. 4: eine weitere schematische Seitenansicht eines erfindungsgemäßen Rohlings.

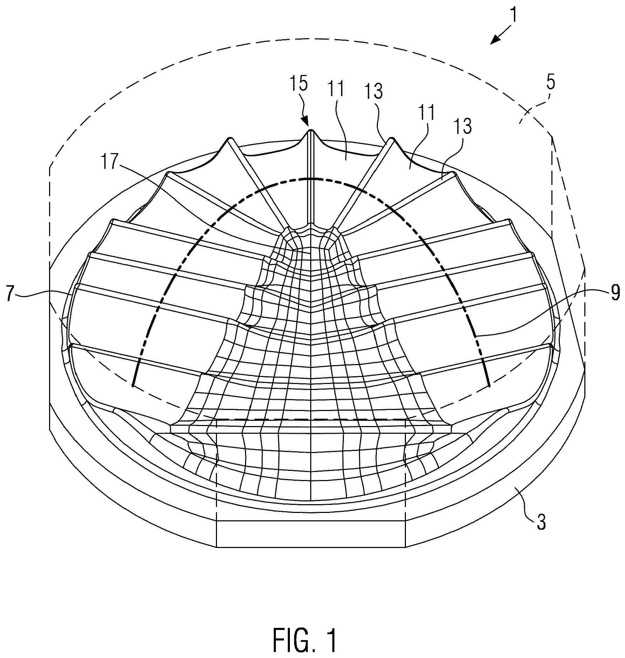

In Fig. 1 ist ein erfindungsgemäßer Rohling 1 für eine dentale Oberkieferprothese dargestellt. Der Rohling 1 ist vom Grundaufbau her scheibenförmig. Er weist eine erste Schicht 3 auf Basis von Zirkonoxidkeramik und eine zweite Schicht 5 auf Basis von Zirkonoxidkeramik auf. Die erste Schicht 3 und die zweite Schicht 5 unterscheiden sich in ihrer Farbe und bilden eine Grenzfläche 7. In der Darstellung gemäß Fig. 1 stellen die Oberseite der ersten Schicht 3 und die Unterseite der zweiten Schicht 5 die Grenzfläche 7 des Rohlings dar. Ein beispielhafter schematischer Verlauf eines Zahnbogens, d.h. der Verlauf der zu erstellenden Zähne der Prothese, ist in Fig. 1 als Linie 9 dargestellt. Im Zahnbogenverlauf 9 ist die Grenzfläche 7 wellenförmig ausgebildet, wobei sich Wellentäler 11 und Wellenberge 13 abwechseln. Im Bereich 15 der zu erstellenden Frontzähne erstrecken sich die Wellenberge 13 in oral-vestibulärer Richtung fächerförmig. Das heißt, dass sich die Kammlinien der Wellenberge 13 von einem Mittelpunkt 17 des Zahnbogens 9 in radialer Richtung, d.h. nach außen, also zur vestibulären Seite hin, in einer Strahlenform erstrecken.

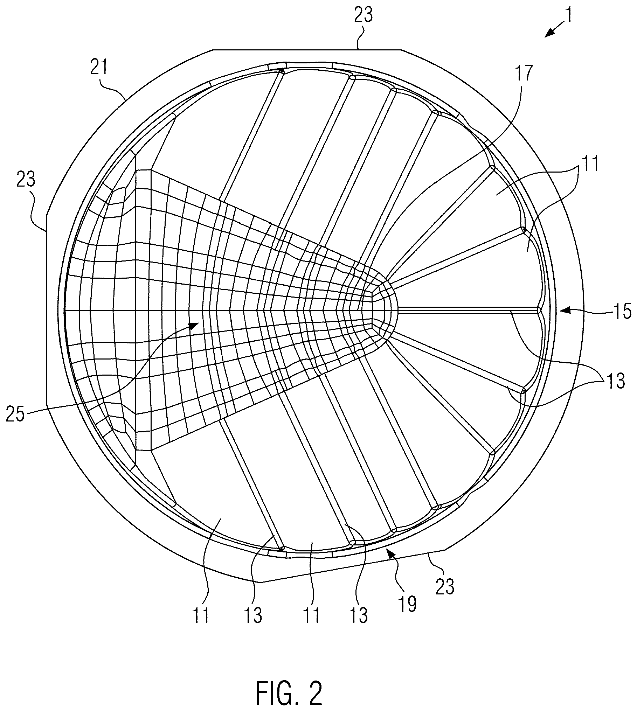

Fig. 2 zeigt eine Draufsicht auf die Oberseite der ersten Schicht 3 des in Fig. 1 dargestellten Rohlings 1. In der Draufsicht ist im Frontzahnbereich 15 der fächerförmige Verlauf der Wellenberge 13 ausgehend vom Strahlenmittelpunkt 17 zu erkennen.

Aus Fig. 2 ist auch ersichtlich, dass die Scheitellinien der Wellentäler 11 und die Wellenberge 13 im Bereich 19 der zu erstellenden Molaren in oral-bukkaler Richtung im Wesentlichen parallel verlaufen. Wie oben beschrieben, wird durch diese Parallelisierung der Wellenbergeberge und Wellentäler im Bereich der Molaren erreicht, dass in der fertigen Dentalprothese auch bei kleinen Zahnbögen eine vergleichsweise große Kaufläche zur Verfügung steht.

Der Rohling gemäß Fig. 2 weist an seinem äußeren Umfang, d.h. Mantelfläche 21, ferner drei Abflachungen 23 auf, die nicht rotationssymmetrisch zueinander angeordnet sind. Die Abflachungen 23 dienen zur Markierung, um die Lage der Wellen- und Strahlengeometrie innerhalb der Scheibe im Verhältnis zur Scheibe an sich zu definieren, so dass die Scheibe bei der Herstellung der Dentalprothese richtig platziert werden kann. Mithin dienen die Abflachungen 23 als Rotationsschutz bzw. Verdrehsicherung beim späteren Fräsvorgang.

Der innere Bereich 25 der ersten Schicht 3 stellt den Bereich der zu erstellenden Prothese, der nach dem Einsetzen der Prothese mit dem Gaumen des Patienten in Berührung kommen würde, d.h. die sog. Gaumenplatte dar. Im Falle von Implantat- bzw. Zahnstumpf-gestützten Prothesen kann der Bereich 25 wegfallen bzw. kann uneingefärbt bleiben.

Fig. 3 zeigt eine Seitenansicht auf den in Fig. 1 dargestellten Rohling 1. Der Rohling weist eine Gesamthöhe 27 auf, die in der konkreten Ausführungsform des in den Figuren dargestellten Rohlings 26,80 mm beträgt. Die Höhe 29 der ersten Schicht beträgt dabei 6,80 mm und die Höhe 31 der zweiten Schicht beträgt 20,00 mm. Im Vergleich zu den aus EP 3 064 170 A1 und EP 3 597 143 A1 bekannten Rohlingen auf Basis von Kunststoffmaterialien, bei denen die Höhe der Gingiva-Schicht und der Zahn-Schicht üblicherweise jeweils etwa 19,00 mm beträgt, konnte die Gesamthöhe des Rohlings 1 deutlich verringert und liegt unterhalb der kritischen Grenze von 30 mm, die eine einfache Bearbeitung in handelsüblichen dentalen Fräsmaschinen ermöglicht. Zudem konnte das Verhältnis von Zahn-Schicht zu Gingiva-Schicht deutlich erhöht werden, wodurch bessere Farb- und Transluzenzverläufe erzielt werden können.

Des Weiteren ist Rohling 1 gemäß Fig. 3 dadurch gekennzeichnet, dass der Winkel 33 zwischen einer fiktiven Gerade 35, die den tiefsten Punkt des Wellentals 11a für den zu erstellenden Zweiten Molaren mit dem tiefsten Punkt des Wellentals 11b für den zu erstellenden mittleren Schneidezahn verbindet, und der Projektion dieser fiktiven Geraden 35 auf eine Grundfläche des Rohlings, hier die Oberseite 37 des Rohlings, 3° beträgt und somit kleiner als der entsprechende Winkel bei aus EP 3 064 170 A1 und EP 3 597 143 A1 bekannten Rohlingen auf Basis von Kunststoffmaterialien ist, der dort üblicherweise 5° beträgt. Durch Verringerung des Winkels 33 kann der Farb- und Transluzenzverlauf von Front- und Backenzähnen der zu erstellenden Prothese weiter verbessert werden.

Fig. 4 zeigt eine weitere Seitenansicht des in Fig. 1 dargestellten Rohlings 1. Der Winkel 39 zwischen der Oberfläche 37 und der Kammlinie der Wellenberge 13a beträgt in der dargestellten Ausführungsform 10° und ist somit deutlich kleiner als der entsprechende Winkel bei aus EP 3 064 170 A1 und EP 3 597 143 A1 bekannten Rohlingen auf Basis von Kunststoffmaterialien ist, der dort üblicherweise 15° beträgt. Die Verringerung des Winkels 39 führt ebenso wie die Verringerung des Winkels 33 zu einer einfacheren Bearbeitung des Rohlings in einer Fräsmaschine, da in diesen Ausführungsformen weniger Hinterschnitte notwendig sind.

## Patentansprüche

1. Rohling (1) für dentale Prothesen, der eine erste Schicht (3) auf Basis von Zirkonoxidkeramik und eine zweite Schicht (5) auf Basis von Zirkonoxidkeramik aufweist, wobei die erste Schicht (3) und die zweite Schicht (5) sich in der Farbe unterscheiden und eine Grenzfläche (7) bilden, wobei die Grenzfläche (7) im Zahnbogenverlauf (9) wellenförmig mit einander abwechselnden Wellentälern (11) und Wellenbergen (13) ausgebildet ist und die Scheitellinien der Wellenberge (13), in der Draufsicht auf die Grenzfläche betrachtet, sich in mesial-distaler Richtung strahlenförmig erstrecken, wobei der Rohling mindestens teilweise kreisbogenförmig ist und der Winkel (33) zwischen einer fiktiven Geraden (35), die den tiefsten Punkt des Wellentals (11a) für den zu erstellenden Zweiten Molaren mit dem tiefsten Punkt des Wellentals (11b) für den zu erstellenden mittleren Schneidezahn verbindet, und der Projektion dieser fiktiven Geraden auf eine Grundfläche (37) des kreisbogenförmigen Rohlings 2,0° bis 4,5° beträgt.

2. Rohling (1) nach Anspruch 1, bei dem die zweite Schicht (5) einen kontinuierlichen oder diskontinuierlichen Farbgradienten aufweist.

3. Rohling (1) nach Anspruch 2, bei dem die Zirkonoxidkeramik der zweiten Schicht (5) Yttrium enthält und der Farbgradient durch einen Gradienten des Gehalts an Yttrium ausgebildet ist.

4. Rohling (1) nach Anspruch 3, bei dem der Gehalt an Yttrium in der zweiten Schicht (5) von der Grenzfläche (7) zu der der Grenzfläche gegenüberliegenden äußeren Oberfläche der zweiten Schicht hin ansteigt.

5. Rohling (1) nach einem der Ansprüche 2 bis 4, bei dem die zweite Schicht (5) eine innere Schicht und eine äußere Schicht aufweist, wobei die innere Schicht an die Grenzfläche (7) zwischen erster und zweiter Schicht angrenzt und die äußere Schicht an die der Grenzfläche gegenüberliegenden äußeren Oberfläche der zweiten Schicht (5) angrenzt.

6. Rohling (1) nach Anspruch 5, bei dem
(a) die innere Schicht einen Yttrium-Gehalt von 2,0 bis 6,0 Mol-%, vorzugsweise von 3,0 bis 5,0 Mol-%, aufweist und
(c) die äußere Schicht einen Yttrium-Gehalt von 3,5 bis 8,0 Mol-%, vorzugsweise von 4,0 bis 7,5 Mol-%, aufweist,
wobei der Yttrium-Gehalt als Anteil der Stoffmenge von Y₂O₃ bezogen auf die Summe der Stoffmengen von Y₂O₃, ZrO₂ und HfO₂ definiert ist.

7. Rohling (1) nach einem der Ansprüche 1 bis 6, bei dem die erste Schicht (3) aus Zirkonoxidkeramik oder einer Mischung von Zirkonoxidkeramik mit einem oder mehreren Materialien ausgewählt aus der Gruppe bestehend aus durch Aluminiumoxid verstärktes Zirkonoxid, durch Zirkonoxid verstärktes Aluminiumoxid, Spinellen, Pigmenten oder Mischungen davon besteht.

8. Rohling (1) nach einem der Ansprüche 1 bis 7, bei dem die Zirkonoxidkeramik der ersten Schicht (3) Erbium und/oder Yttrium enthält, wobei vorzugsweise die erste Schicht einen Erbium-Gehalt von 0,0 bis 4,5 Mol-%, insbesondere 0,5 bis 4,25 Mol-%, besonders bevorzugt 1,5 bis 3,5 Mol.-%, wobei der Erbium-Gehalt als Anteil der Stoffmenge von Er₂O₃ bezogen auf die Summe der Stoffmengen von Er₂O₃, ZrO₂ und HfO₂ definiert ist, und/oder einen Yttrium-Gehalt von 0,0 bis 4,5 Mol-%, insbesondere 0,5 bis 4,25 Mol-%, besonders bevorzugt 0,75 bis 2,0 Mol.-%, aufweist, wobei der Yttrium-Gehalt als Anteil der Stoffmenge von Y₂O₃ bezogen auf die Summe der Stoffmengen von Y₂O₃, ZrO₂ und HfO₂ definiert ist, wobei ferner besonders bevorzugt die Summe des Erbium- und Yttrium-Gehalts 1,5 bis 6,0 Mol.%, insbesondere 2,0 bis 4,5 Mol.%, besonders bevorzugt 2,5 bis 4,0 Mol-% beträgt.

9. Rohling (1) nach einem der Ansprüche 1 bis 8, bei dem die erste Schicht (3) weißlich oder rosafarben und die zweite Schicht (5) zahnfarben ist.

10. Rohling (1) nach einem der Ansprüche 1 bis 9, bei dem die Zirkonoxidkeramik der ersten Schicht (3) und die Zirkonoxidkeramik der zweiten Schicht (5) vorgesintert sind, wobei die Zirkonoxidkeramik der ersten Schicht (3) und die Zirkonoxidkeramik der zweiten Schicht (5) unabhängig voneinander vorzugsweise eine Dichte von 1,8 bis 4,4 g/cm³, insbesondere 2,5 bis 4,0 g/cm³, aufweisen.

11. Rohling (1) nach einem der Ansprüche 1 bis 10, bei dem die erste Schicht (3) und die zweite Schicht (5) durch einstückige Herstellung miteinander verbunden sind.

12. Rohling (1) nach einem der Ansprüche 1 bis 11, der die Form einer Scheibe, insbesondere die Form einer kreisrunden Scheibe hat.

13. Rohling (1) nach Anspruch 12, bei dem am Außenumfang des Rohlings (1) ein Vorsprung, vorzugsweise ein nach außen vorspringender Einspannrand, ausgebildet ist.

14. Rohling (1) nach einem der Ansprüche 1 bis 13, bei dem die Grenzfläche (7), in der Draufsicht auf die Grenzfläche betrachtet, im Bereich (15) der zu erstellenden Frontzähne in oral-vestibulärer Richtung fächerförmig verlaufende Scheitellinien der Wellenberge aufweist.

15. Rohling (1) nach einem der Ansprüche 1 bis 14, bei dem die Grenzfläche (7), in der Draufsicht auf die Grenzfläche betrachtet, im Bereich (19) der zu erstellenden Molaren in oral-bukkaler Richtung strahlenförmige Scheitellinien der Wellentäler oder zueinander im Wesentlichen parallele Scheitellinien der Wellentäler aufweist.

16. Rohling (1) nach Anspruch 1 bis 15, bei dem der Winkel zwischen einer fiktiven Geraden (35), die den tiefsten Punkt des Wellentals (11a) für den zu erstellenden Zweiten Molaren mit dem tiefsten Punkt des Wellentals (11b) für den zu erstellenden mittleren Schneidezahn verbindet, und der Projektion dieser fiktiven Geraden auf eine Grundfläche (37) des kreisbogenförmigen Rohlings 2,0° bis 4,0°, besonders bevorzugt 2,5° bis 3,5° und am meisten bevorzugt etwa 3,0° beträgt.

17. Rohling (1) nach einem der Ansprüche 1 bis 16, bei dem die Höhe (27) des Rohlings nicht mehr als 30 mm, vorzugsweise 20 bis 30 mm, besonders bevorzugt 25 bis 29 mm, beträgt, wobei vorzugsweise (i) die Höhe (29) der ersten Schicht (3) 5,0 bis 9,0 mm, insbesondere 6,0 bis 7,5 mm, besonders bevorzugt etwa 7,0 mm, beträgt und/oder (ii) die Höhe (31) der zweiten Schicht (5) 15,0 bis 25,0 mm, insbesondere 18,0 bis 22,0 mm, besonders bevorzugt etwa 20 mm, beträgt.

18. Rohling (1) nach einem der Ansprüche 1 bis 17, bei dem der Winkel (39) zwischen einer Grundfläche (37) des kreisbogenförmigen Rohlings und der Kammlinie der Wellenberge (13a) der wellenförmigen Grenzfläche 7° bis 13°, vorzugsweise 9° bis 11°, und besonders bevorzugt etwa 10°, beträgt.

19. Verfahren zur Herstellung eines Rohlings (1) nach einem der Ansprüche 1 bis 18, bei dem
(i) ein erster und ein zweiter Grünkörper bereitgestellt werden, wobei der erste Grünkörper zur Bildung der ersten Schicht (3) des Rohlings (1) und der zweite Grünkörper zur Bildung der zweiten Schicht (5) des Rohlings (1) vorgesehen sind, und wobei eine erste Seite des ersten Grünkörpers die aus Wellental (11) und Wellenberg (13) gebildete Wellengeometrie der ersten Schicht (3) aufweist und eine erste Seite des zweiten Grünkörpers die entsprechende inverse Wellengeometrie aufweist;
(ii) der erste und zweite Grünkörper übereinandergelegt werden, so dass die erste Seite des ersten Grünkörpers und die erste Seite des zweiten Grünkörpers vollflächig in engen Kontakt miteinander kommen;
(iii) der erste und zweite Grünkörper zusammengepresst werden; und
(iv) mindestens eine Wärmebehandlung durchgeführt wird, um das Zirkonoxid in vorgesintertes Zirkonoxid umzuwandeln.

20. Verfahren nach Anspruch 19, bei dem in Schritt (iii) der erste und zweite Grünkörper isostatisch bei einem Druck von mehr als 100 MPa, vorzugsweise bei einem Druck von 150 bis 1000 MPa, bevorzugt 150 bis 500 MPa, zusammengepresst werden.

21. Verfahren nach Anspruch 19 oder 20, bei dem in Schritt (iv) die Wärmebehandlung bei einer Temperatur von 700 bis 1200°C und vorzugsweise bei einer Temperatur von 800 bis 1100°C stattfindet.

22. Verwendung eines Rohlings (1) nach einem der Ansprüche 1 bis 18 zur Herstellung einer Dentalrestauration und insbesondere einer Dentalprothese, wobei die Dentalprothese vorzugsweise ausgewählt ist aus der Gruppe bestehend aus Oberkiefer-Totalprothesen, Unterkiefer-Totalprothesen, Oberkiefer-Teilprothesen und Unterkiefer-Teilprothesen.

23. Verfahren zur Herstellung einer Dentalrestauration, bei dem
(i-1) einem Rohling (1) gemäß einem der Ansprüche 1 bis 18 durch maschinelle Bearbeitung die Form der Dentalrestauration gegeben wird,
(i-2) mindestens eine Wärmebehandlung durchgeführt wird, um das Zirkonoxid der ersten und zweiten Schicht in dichtgesintertes Zirkonoxid umzuwandeln, und
(i-3) gegebenenfalls die Oberfläche der erhaltenen Dentalrestauration endbehandelt wird.

24. Verfahren nach Anspruch 23, bei dem die maschinelle Bearbeitung mit computergesteuerten Fräs- und/oder Schleifvorrichtungen erfolgt.

25. Verfahren nach Anspruch 23 oder 24, bei dem die Dentalrestauration ausgewählt ist aus der Gruppe bestehend aus Oberkiefer-Totalprothesen, Unterkiefer-Totalprothesen, Oberkiefer-Teilprothesen und Unterkiefer-Teilprothesen.

## Claims

1. Blank (1) for dental prostheses, which has a first layer (3) based on zirconium oxide ceramic and a second layer (5) based on zirconium oxide ceramic, the first layer (3) and the second layer (5) differing in color and forming a boundary surface (7), wherein the boundary surface (7) is formed in the course of the dental arch (9) in an undulating shape with alternating wave troughs (11) and wave crests (13), and the vertex lines of the wave crests (13), viewed in plan view of the boundary surface, extend radially in the mesial-distal direction, wherein the blank is at least partially circular-arc-shaped and the angle (33) between a fictitious straight line (35) connecting the lowest point of the wave trough (11a) for the second molar to be produced with the lowest point of the wave trough (11b) for the central incisor to be produced, and the projection of this fictitious straight line onto a base surface (37) of the circular-arc-shaped blank is 2.0° to 4.5°.

2. Blank (1) according to claim 1, wherein the second layer (5) has a continuous or discontinuous color gradient.

3. Blank (1) according to claim 2, wherein the zirconium oxide ceramic of the second layer (5) comprises yttrium and the color gradient is formed by a gradient of the content of yttrium.

4. Blank (1) according to claim 3, wherein the content of yttrium in the second layer (5) increases from the boundary surface (7) to the outer surface of the second layer opposite the boundary surface.

5. Blank (1) according to any one of claims 2 to 4, wherein the second layer (5) comprises an inner layer and an outer layer, the inner layer being adjacent to the boundary surface (7) between the first and second layers, and the outer layer being adjacent the outer surface of the second layer (5) opposite the boundary surface.

6. Blank (1) according to claim 5, wherein
(a) the inner layer has an yttrium content of from 2.0 to 6.0 mol%, preferably from 3.0 to 5.0 mol%, and
(c) the outer layer has an yttrium content of from 3.5 to 8.0 mol%, preferably from 4.0 to 7.5 mol%,
wherein the yttrium content is defined as the proportion of the amount of substance of Y₂O₃ relative to the sum of the amounts of substance of Y₂O₃, ZrO₂, and HfO₂.

7. Blank (1) according to any one of claims 1 to 6, wherein the first layer (3) is made of zirconium oxide ceramic or a mixture of zirconium oxide ceramic with one or more materials selected from the group consisting of alumina-reinforced zirconium oxide, zirconium oxide reinforced alumina, spinels, pigments, or mixtures thereof.

8. Blank (1) according to any one of claims 1 to 7, wherein the zirconium oxide ceramic of the first layer (3) comprises erbium and/or yttrium, the first layer preferably having an erbium content of 0.0 to 4.5 mol%, in particular 0.5 to 4.25 mol%, particularly preferably 1.5 to 3.5 mol%, wherein the erbium content is defined as the proportion of the amount of substance of Er₂O₃ relative to the sum of the amounts of substance of Er₂O₃, ZrO2, and HfO₂, and/or an yttrium content of 0.0 to 4.5 mol%, in particular 0.5 to 4.25 mol%, particularly preferably 0.75 to 2.0 mol.%, wherein the yttrium content is defined as the proportion of the amount of substance of Y₂O₃ relative to the sum of the amounts of substance of Y₂O₃, ZrO₂ and HfO₂, the sum of the erbium and yttrium content being furthermore particularly preferred 1.5 to 6.0 mol%, in particular 2.0 to 4.5 mol%, particularly preferred 2.5 to 4.0 mol%.

9. Blank (1) according to any one of claims 1 to 8, wherein the first layer (3) is whitish or pinkish and the second layer (5) is tooth-colored.

10. Blank (1) according to any one of claims 1 to 9, wherein the zirconium oxide ceramic of the first layer (3) and the zirconium oxide ceramic of the second layer (5) are pre-sintered, wherein the zirconium oxide ceramic of the first layer (3) and the zirconium oxide ceramic of the second layer (5) independently of one each other, preferably have a density of 1.8 to 4.4 g/cm³, in particular 2.5 to 4.0 g/cm³.

11. Blank (1) according to any one of claims 1 to 10, wherein the first layer (3) and the second layer (5) are connected to each other by integral manufacture.

12. Blank (1) according to any one of claims 1 to 11, which has the shape of a disc, in particular the shape of a circular disc.

13. Blank (1) according to claim 12, wherein a protrusion, preferably an outwardly projecting clamping edge, is formed on the outer circumference of the blank (1).

14. Blank (1) according to one of claims 1 to 13, wherein the boundary surface (7), viewed in plan view of the boundary surface, in the region (15) of the anterior teeth to be produced has, in the oral-vestibular direction, vertex lines of wave crests in a fan shape.

15. Blanks (1) according to any of claims 1 to 14, wherein the boundary surface (7), viewed in plan view of the boundary surface, in the region (19) of the molars to be produced, has in the oral-buccal direction radiating crest lines of the wave troughs or vertex lines of the wave troughs which are substantially parallel to one another.

16. Blank (1) according to claims 1 to 15, wherein the angle between a fictitious straight line (35) connecting the lowest point of the wave trough (11a) for the second molar to be produced with the lowest point of the wave trough (11b) for the central incisor to be produced, and the projection of this fictitious straight line onto a base surface (37) of the circular arc-shaped blank is 2.0° to 4.0°, particularly preferably 2.5° to 3.5°, and most preferably about 3.0° .

17. Blank (1) according to one of claims 1 to 16, wherein the height (27) of the blank is not more than 30 mm, preferably 20 to 30 mm, more preferably 25 to 29 mm, wherein preferably (i) the height (29) of the first layer (3) is 5.0 to 9.0 mm, in particular 6.0 to 7.5 mm, more preferably about 7.0 mm, and/or (ii) the height (31) of the second layer (5) is 15.0 to 25.0 mm, in particular 18.0 to 22.0 mm, more preferably about 20 mm.

18. Blank (1) according to one of claims 1 to 17, wherein the angle (39) between a base surface (37) of the circular arc-shaped blank and the vertex line of the wave crests (13a) of the undulating boundary surface is 7° to 13°, preferably 9° to 11°, and more preferably about 10°.

19. Process for preparing a blank (1) according to any one of claims 1 to 18, comprising
(i) providing a first and a second green body, wherein the first green body is provided for forming the first layer (3) of the blank (1) and the second green body is provided for forming the second layer (5) of the blank (1), and wherein a first side of the first green body has the wave geometry of the first layer **(3)** formed by wave trough (11) and wave crests (13), and a first side of the second green body has the corresponding inverse wave geometry;
(ii) putting the first and second green bodies on top of each other so that the first side of the first green body and the first side of the second green body come into close contact with each other over their entire surface;
(iii) pressing the first and second green bodies together; and
(iv) carrying out at least one heat treatment to convert the zirconium oxide into pre-sintered zirconium oxide.

20. The process according to claim 19, wherein in step (iii) the first and second green bodies are isostatically compressed together at a pressure of more than 100 MPa, preferably at a pressure of 150 to 1000 MPa, preferably 150 to 500 MPa.

21. Process according to claim 19 or 20, wherein in step (iv) the heat treatment takes place at a temperature of from 700 to 1200°C and preferably at a temperature of from 800 to 1100°C.

22. Use of a blank (1) according to one of claims 1 to 18 for preparing a dental restoration and, in particular, a dental prosthesis, wherein the dental prosthesis is preferably selected from the group consisting of maxillary full dentures, mandibular full dentures, maxillary partial dentures, and mandibular partial dentures.

23. Process for preparing a dental restoration, comprising
(i-1) providing the shape of the dental restoration to a blank (1) according to any one of claims 1 to 18 by machining,
(i-2) carrying out at least one heat treatment to convert the zirconium oxide of the first and second layers into densely sintered zirconium oxide, and
(i-3) optionally, finishing the surface of the dental restoration obtained.

24. Process according to claim 23, wherein the machining is carried out with computer-controlled milling and/or grinding devices.

25. Process according to claim 23 or 24, wherein the dental restoration is selected from the group consisting of maxillary full dentures, mandibular full dentures, maxillary partial dentures, and mandibular partial dentures.

## Revendications

1. Ébauche (1) de prothèse dentaire, qui présente une première couche (3) à base de céramique d'oxyde de zirconium et une deuxième couche (5) à base de céramique d'oxyde de zirconium, dans laquelle la première couche (3) et la deuxième couche (5) se distinguent par la couleur et forment une interface (7), dans laquelle l'interface (7) est réalisée, dans le tracé de l'arcade dentaire (9), sous la forme d'ondulations avec des creux d'ondulation (11) et des crêtes d'ondulation (13) en alternance et les lignes de sommet des crêtes d'ondulation (13) s'étendent, vues dans la vue de dessus sur l'interface, en forme de rayon dans la direction mésiale-distale, dans laquelle l'ébauche est au moins en partie en forme d'arc de cercle et l'angle (33) entre une ligne droite fictive (35), qui relie le point le plus bas du creux d'ondulation (11a) pour la deuxième molaire à créer au point le plus bas du creux d'ondulation (11b) pour l'incisive médiane à créer, et la projection de ladite ligne droite fictive sur une surface de base (37) de l'ébauche en forme d'arc de cercle est compris entre 2,0° et 4,5°.

2. Ébauche (1) selon la revendication 1, dans laquelle la deuxième couche (5) présente un gradient de couleur continu ou discontinu.

3. Ébauche (1) selon la revendication 2, dans laquelle la céramique d'oxyde de zirconium de la deuxième couche (5) contient de l'yttrium et le gradient de couleur est réalisé par un gradient de la teneur en yttrium.

4. Ébauche (1) selon la revendication 3, dans laquelle la teneur en yttrium dans la deuxième couche (5) augmente depuis l'interface (7) vers la surface extérieure de la deuxième couche opposée à l'interface.

5. Ébauche (1) selon l'une quelconque des revendications 2 à 4, dans laquelle la deuxième couche (5) présente une couche intérieure et une couche extérieure, dans laquelle la couche intérieure jouxte l'interface (7) entre la première et la deuxième couche et la couche extérieure jouxte la surface extérieure de la deuxième couche (5) faisant face à l'interface.

6. Ébauche (1) selon la revendication 5, dans laquelle
(a) la couche intérieure présente une teneur en yttrium de 2,0 à 6,0 % mol, de préférence de 3,0 à 5,0 % mol, et
(c) la couche extérieure présente une teneur en yttrium de 3,5 à 8,0 % mol, de préférence de 4,0 à 7,5 % mol,
dans laquelle la teneur en yttrium est définie comme la proportion de la quantité de matière de Y₂O₃ par rapport à la somme des quantités de matière de Y₂O₃, ZrO₂ et HfO₂.

7. Ébauche (1) selon l'une quelconque des revendications 1 à 6, dans laquelle la première couche (3) est constituée de céramique d'oxyde de zirconium ou d'un mélange de céramique d'oxyde de zirconium avec un ou plusieurs matériaux choisis parmi le groupe constitué d'oxyde de zirconium renforcé par de l'oxyde d'aluminium, d'oxyde d'aluminium renforcé par de l'oxyde de zirconium, de spinelles, de pigments ou de mélanges de ceux-ci.

8. Ébauche (1) selon l'une quelconque des revendications 1 à 7, dans laquelle la céramique d'oxyde de zirconium de la première couche (3) contient de l'erbium et/ou de l'yttrium, dans laquelle de préférence la première couche présente une teneur en erbium de 0,0 à 4,5 % mol, en particulier de 0,5 à 4,25 % mol, de manière particulièrement préférée de 1,5 à 3,5 % mol, dans laquelle la teneur en erbium est définie comme une proportion de la quantité de matière d'Er₂O₃ par rapport à la somme des quantités de matières d'Er₂O₃, ZrO₂ et HfO₂, et/ou présente une teneur en yttrium de 0,0 à 4,5 % en mol, en particulier de 0,5 à 4,25 % en mol, de manière particulièrement préférée de 0,75 à 2,0 % en mol, dans laquelle la teneur en yttrium est définie comme une proportion de la quantité de matière de Y₂O₃ par rapport à la somme des quantités de matière de Y₂O₃, ZrO₂ et HfO₂, dans laquelle, en outre, de manière particulièrement préférée la somme de la teneur en erbium et en yttrium va de 1,5 à 6,0 % en mol, en particulier de 2,0 à 4,5 % en mol, de manière particulièrement préférée de 2,5 à 4,0 % en mol.

9. Ébauche (1) selon l'une quelconque des revendications 1 à 8, dans laquelle la première couche (3) est blanche ou rose et la deuxième couche (5) est présente une teinte de dent.

10. Ébauche (1) selon l'une quelconque des revendications 1 à 9, dans laquelle la céramique d'oxyde de zirconium de la première couche (3) et la céramique d'oxyde de zirconium de la deuxième couche (5) sont préfrittées, dans laquelle la céramique d'oxyde de zirconium de la première couche (3) et la céramique d'oxyde de zirconium de la deuxième couche (5) présentent de préférence indépendamment l'une de l'autre une densité de 1,8 à 4,4 g/cm³, en particulier de 2,5 à 4,0 g/cm³.

11. Ébauche (1) selon l'une quelconque des revendications 1 à 10, dans laquelle la première couche (3) et la deuxième couche (5) sont reliées entre elles par fabrication monobloc.

12. Ébauche (1) selon l'une quelconque des revendications 1 à 11, qui présente la forme d'un disque, en particulier la forme d'un disque rond circulaire.

13. Ébauche (1) selon la revendication 12, dans laquelle une saillie, de préférence un bord de serrage faisant saillie vers l'extérieur, est réalisée sur la périphérie extérieure de l'ébauche (1).

14. Ébauche (1) selon l'une quelconque des revendications 1 à 13, dans laquelle l'interface (7) présente, dans la vue de dessus sur l'interface, des lignes de sommet des crêtes d'ondulation s'étendant en éventail dans la zone (15) des dents antérieures à créer dans la direction linguale-vestibulaire.

15. Ébauche (1) selon l'une quelconque des revendications 1 à 14, dans laquelle l'interface (7) présente, dans la vue de dessus sur l'interface, dans la zone (19) des molaires à créer, des lignes de sommet des creux d'ondulation en forme de rayons dans la direction linguale-buccale ou des lignes de sommet des creux d'ondulation sensiblement parallèles les unes par rapport aux autres.

16. Ébauche (1) selon la revendication 1 à 15, dans laquelle l'angle entre une ligne droite fictive (35), qui relie le point le plus bas du creux d'ondulation (11a) pour la deuxième molaire à créer au point le plus bas du creux d'ondulation (11b) pour l'incisive centrale à créer, et la projection de ladite ligne droite fictive sur une surface de base (37) de l'ébauche en forme d'arc de cercle va de 2,0° à 4,0°, de manière particulièrement préférée de 2,5° à 3,5° et idéalement est d'environ 3,0°.

17. Ébauche (1) selon l'une quelconque des revendications 1 à 16, dans laquelle la hauteur (27) de l'ébauche n'est pas supérieure à 30 mm, de préférence va de 20 à 30 mm, de manière particulièrement préférée va de 25 à 29 mm, dans laquelle de préférence (i) la hauteur (29) de la première couche (3) va de 5,0 à 9,0 mm, en particulier de 6,0 à 7,5 mm, de manière particulièrement préférée est environ de 7,0 mm, et/ou (ii) la hauteur (31) de la deuxième couche (5) va de 15,0 à 25,0 mm, en particulier de 18,0 à 22,0 mm, de manière particulièrement préférée est d'environ 20 mm.

18. Ébauche (1) selon l'une quelconque des revendications 1 à 17, dans laquelle l'angle (39) entre une surface de base (37) de l'ébauche en forme d'arc de cercle et la ligne de crête des crêtes d'ondulation (13a) de l'interface en forme d'ondulation va de 7° à 13°, de préférence de 9° à 11°, et de manière particulièrement préférée est d'environ 10°.

19. Procédé de fabrication d'une ébauche (1) selon l'une quelconque des revendications 1 à 18, dans lequel
(i) un premier et un deuxième corps vert sont fournis, dans lequel le premier corps vert est prévu pour former la première couche (3) de l'ébauche (1) et le deuxième corps vert est prévu pour former la deuxième couche (5) de l'ébauche (1), et dans lequel un premier côté du premier corps vert présente la géométrie d'ondulation de la première couche (3) formée à partir du creux d'ondulation (11) et de la crête d'ondulation (13) et un premier côté du deuxième corps vert présente la géométrie d'ondulation inverse correspondante ;
(ii) le premier et le deuxième corps vert sont superposés de telle sorte que le premier côté du premier corps vert et le premier côté du deuxième corps vert sont en contact étroit sur toute la surface ;
(iii) les premier et deuxième corps verts sont comprimés ; et
(iv) au moins un traitement thermique est effectué pour transformer l'oxyde de zirconium en oxyde de zirconium préfritté.

20. Procédé selon la revendication 19, dans lequel dans l'étape (iii), le premier et le deuxième corps vert sont comprimés isostatiquement à une pression supérieure à 100 MPa, de préférence à une pression de 150 à 1000 MPa, de préférence de 150 à 500 MPa.

21. Procédé selon la revendication 19 ou 20, dans lequel dans l'étape (iv), le traitement thermique a lieu à une température de 700 à 1200 °C et de préférence à une température de 800 à 1100 °C.

22. Utilisation d'une ébauche (1) selon l'une quelconque des revendications 1 à 18 pour la fabrication d'une restauration dentaire et en particulier d'une prothèse dentaire, dans laquelle la prothèse dentaire est de préférence choisie parmi le groupe constitué de prothèses totales mâchoire supérieure, de prothèses totales de mâchoire inférieure, de prothèses partielles de mâchoire supérieure et de prothèses partielles de mâchoire inférieure.

23. Procédé de fabrication d'une restauration dentaire, dans lequel
(i-1) une ébauche (1) selon l'une quelconque des revendications 1 à 18 se voit donner la forme d'une restauration dentaire par usinage mécanique,
(i-2) au moins un traitement thermique est effectué pour convertir l'oxyde de zirconium des première et deuxième couches en oxyde de zirconium fritté densément, et
(i-3) le cas échéant, la surface de la restauration dentaire obtenue est soumise à un traitement de finition.

24. Procédé selon la revendication 23, dans lequel l'usinage mécanique est effectué avec des dispositifs de fraisage et/ou de rectification commandés par ordinateur.

25. Procédé selon la revendication 23 ou 24, dans lequel la restauration dentaire est choisie parmi le groupe constitué de prothèses totales de mâchoire supérieure, de prothèses totales de mâchoire inférieure, de prothèses partielles de mâchoire supérieure et de prothèses partielles de mâchoire inférieure.
